# EUROPEAN PATENT APPLICATION

(11) **EP 2 960 339 A1**
(43) Date of publication of application: **30.12.2015**
(21) Application number: 14174714.7
(22) Date of filing: 27.06.2014
(51) Int. Cl.: C12Q 1/48

(54) **Method and kit for the identification of glycosyl transferase substrates**

(71) Applicant: Technische Universität München, 80333 München (DE)
(72) Inventor: Schwab, Wilfried, 97828 Marktheidenfeld (DE)
(74) Representative: Engelhard, Markus

(57) **Abstract**

The present invention relates to a method for identifying a substrate of a glycosyl transferase of a plant, and to a kit comprising a glycosyl transferase of a plant and an aglycone library from said plant.

SEQUENCE LISTING

<110>  Technische Universität München

<120>  Method and Kit for the Identification of Glycosyl Transferase
       Substrates

<130>  U30569EP

<160>  24

<170>  PatentIn version 3.5

<210>  1
<211>  473
<212>  PRT
<213>  Vitis vinifera

<400>  1

Met Gly Ser Met Glu Lys Pro His Ala Val Cys Ile Pro Tyr Pro Ala
1               5                   10                  15

Gln Gly His Ile Asn Pro Met Leu Lys Val Ala Lys Leu Leu His Phe
            20                  25                  30

Arg Gly Phe Arg Ile Thr Phe Val Asn Thr Glu Phe Asn His Thr Arg
        35                  40                  45

Leu Leu Lys Ala Gln Gly Pro Asn Ser Leu Asn Gly Leu Pro Thr Phe
    50                  55                  60

Gln Phe Glu Thr Ile Pro Asp Gly Leu Pro Pro Ser Asn Val Asp Ala
65                  70                  75                  80

Thr Gln Asp Ile Pro Ser Leu Cys Ala Ser Thr Lys Lys Asn Cys Leu
                85                  90                  95

Ala Pro Phe Arg Arg Leu Leu Ala Lys Leu Asn Asp Arg Gly Pro Pro
            100                 105                 110

Val Thr Cys Ile Phe Ser Asp Ala Val Met Ser Phe Thr Leu Asp Ala
        115                 120                 125

Ala Gln Glu Leu Gly Ile Pro Asp Leu Leu Leu Trp Thr Ala Ser Ala
    130                 135                 140

Cys Gly Phe Met Ala Tyr Val Gln Tyr Arg Ser Leu Ile Asp Lys Gly
145                 150                 155                 160

Phe Thr Pro Leu Lys Asp Glu Ser Tyr Leu Thr Asn Gly Tyr Leu Asp
                165                 170                 175

Thr Val Val Asp Trp Ile Pro Gly Met Lys Gly Ile Arg Leu Lys Asp
            180                 185                 190

Leu Pro Ser Phe Ile Arg Thr Thr Asp Pro Asp Asp Ile Met Leu Asp
        195                 200                 205

Phe Ala Met Gly Glu Leu Glu Arg Ala Arg Lys Ala Ser Ala Ile Ile
    210                 215                 220

Phe Asn Thr Phe Asp Ala Leu Glu Gln Glu Val Leu Asp Ala Ile Ala
225                 230                 235                 240

Pro Met Tyr Pro Pro Ile Tyr Thr Ile Gly Pro Leu Gln Leu Leu Pro
                245                 250                 255

Asp Gln Ile His Asp Ser Glu Leu Lys Leu Ile Gly Ser Asn Leu Trp
            260                 265                 270

Lys Glu Glu Pro Glu Cys Leu Lys Trp Leu Asp Ser Lys Glu Pro Asn
        275                 280                 285

Ser Val Val Tyr Val Asn Tyr Gly Ser Ile Thr Val Met Thr Pro Gln
    290                 295                 300

Gln Leu Ile Glu Phe Ala Trp Gly Leu Ala Asn Ser Asn Gln Ser Phe
305                 310                 315                 320

Leu Trp Ile Leu Arg Pro Asp Leu Val Ser Gly Glu Ser Ala Ile Leu
                325                 330                 335

Pro Pro Glu Phe Val Ala Glu Thr Glu Asp Arg Gly Leu Leu Ala Gly
            340                 345                 350

Trp Cys Pro Gln Glu Gln Val Leu Thr His Gln Ala Ile Gly Gly Phe
        355                 360                 365

Leu Thr His Asn Gly Trp Asn Ser Thr Ile Glu Gly Leu Cys Ala Gly
    370                 375                 380

Val Pro Met Ile Cys Trp Pro Phe Phe Ala Glu Gln Gln Thr Asn Cys
385                 390                 395                 400

Arg Tyr Cys Cys Thr Glu Trp Gly Val Gly Met Glu Ile Asp Ser Asp
                405                 410                 415

Val Lys Arg Asp Glu Val Ala Lys Leu Val Arg Glu Leu Met Val Gly
        420                 425                 430

Glu Lys Gly Lys Val Met Lys Lys Lys Thr Met Glu Trp Lys His Arg
            435                 440                 445

Ala Glu Val Ala Thr Thr Gly Pro Asp Gly Ser Ser Tyr Leu Asn Leu
    450                 455                 460

Glu Lys Ile Phe Glu Gln Val Leu Leu
465                 470

<210>  2
<211>  442
<212>  PRT
<213>  Vitis vinifera

<400>  2

Met Pro Arg Arg Val Val Leu Val Pro Cys Pro Phe Gln Gly His Ile
1               5                   10                  15

Asn Pro Met Leu Gln Leu Gly Ala Ile Leu His Ser Arg Gly Phe Ser
            20                  25                  30

Ile Thr Val Ala His Thr Gln Tyr Asn Ser Pro Asp Pro Ser Asn His
        35                  40                  45

Pro Asp Phe Ser Phe Leu Pro Ile Pro Asp Gly Leu Ser Asp Gly Gln
    50                  55                  60

Asn Phe Ala Ser Leu Leu Asn Leu Val Leu Ala Ala Asn Val Asn Cys
65                  70                  75                  80

Glu Ser Pro Leu Arg Glu Tyr Leu Ala Glu Lys Gln Glu Gln His Gly
                85                  90                  95

Asp Ile Ala Cys Ile Ile His Asp Ile Thr Met Tyr Phe Ala Glu Ala
            100                 105                 110

Val Ala Asn His Leu Lys Val Pro Ser Ile Asn Leu Val Thr Ser Asn
        115                 120                 125

Val Ser Thr Thr Ile Ala His Asn Ala Phe Pro Ser Leu Leu Glu Lys
    130                 135                 140

Gly His Ile Pro Leu Gln Gly Ser Thr Leu His Asp Pro Val Pro Glu
145                 150                 155                 160

Leu His Pro Leu Arg Phe Lys Asp Leu Pro Ile Ser Arg Leu Gly Asp
                165                 170                 175

Leu Glu Val Phe Phe Gln Ile Leu Val Asn Met Tyr Lys Lys Lys Phe
            180                 185                 190

Ser Ser Pro Ile Ile Trp Asn Thr Met Asp Cys Leu Glu Gln Ser Ser
        195                 200                 205

Leu Thr Gln Arg Gln Gln Gln Leu Gln Val Pro Phe Phe Pro Ile Gly
    210                 215                 220

Pro Leu His Lys Leu Ala Pro Pro Ser Ser Ser Ser Leu Leu Glu Glu
225                 230                 235                 240

Asp Ser Ser Cys Ile Thr Trp Leu Asp Lys Gln Ser Pro Lys Ser Val
                245                 250                 255

Ile Tyr Val Ser Trp Gly Ser Leu Ala Cys Met Asp Ala Lys Asp Leu
            260                 265                 270

Ala Glu Val Ala Trp Gly Leu Ala Asn Ser Asn Gln Pro Phe Leu Trp
        275                 280                 285

Val Val Arg Pro Gly Ser Val Arg Gly Ser Gln Trp Ile Glu Gln Leu
    290                 295                 300

Pro Glu Thr Phe Met Asp Thr Val Gly Glu Arg Cys His Ile Val Lys
305                 310                 315                 320

Trp Ala Pro Gln Lys Glu Val Leu Gly His Arg Ala Val Gly Gly Phe
                325                 330                 335

Trp Ser His Cys Gly Trp Asn Ser Thr Leu Glu Ser Ile Ser Glu Gly
            340                 345                 350

Val Pro Met Ile Cys Arg Pro Tyr Ser Gly Asp Gln Arg Val Asn Thr
        355                 360                 365

Arg Tyr Ile Ser His Val Trp Lys Val Gly Leu Glu Leu Glu Ser Asp
    370                 375                 380

Glu Leu Glu Arg Val Glu Ile Glu Arg Ala Val Arg Arg Leu Met Val
385                 390                 395                 400

Asp Gly Glu Gly Glu Glu Met Arg Gln Arg Ala Met Glu Leu Lys Glu
                405                 410                 415

Lys Val Glu Ile Cys Thr Ser Glu Gly Gly Ser Ser Asn Arg Ala Leu
            420                 425                 430

Lys Glu Leu Val Glu Tyr Ile Ser Ser Phe
        435                 440

<210>  3
<211>  38
<212>  DNA
<213>  Artificial Sequence

<220>
<223>  VvActin forward primer

<400>  3
aggtgacact atagaatact tgcatccctc agcacctt                              38

<210>  4
<211>  39
<212>  DNA
<213>  Artificial Sequence

<220>
<223>  VvActin reverse primer

<400>  4
gtacgactca ctatagggat cctgtggaca atggatgga                             39

<210>  5
<211>  41
<212>  DNA
<213>  Artificial Sequence

<220>
<223>  VvAP47 forward primer

<400>  5
aggtgacact atagaatatg ttgttgaggc tgttgcgctg t                          41

<210>  6
<211>  41
<212>  DNA
<213>  Artificial Sequence

<220>
<223>  VvAP47 reverse primer

<400>  6
gtacgactca ctatagggaa ggccctctct cctccaacca a                          41

<210>  7
<211>  38
<212>  DNA
<213>  Artificial Sequence

<220>

<223>  VvPP2A forward primer

<400>  7
aggtgacact atagaataag ggttgtgcca cactgggc                              38


<210>  8
<211>  41
<212>  DNA
<213>  Artificial Sequence

<220>
<223>  VvPP2A reverse primer

<400>  8
gtacgactca ctatagggag caaccatgta gcgaacacgc c                          41


<210>  9
<211>  41
<212>  DNA
<213>  Artificial Sequence

<220>
<223>  VvSAND forward primer

<400>  9
aggtgacact atagaataac ccctttgctc ggaggaacag a                          41


<210>  10
<211>  40
<212>  DNA
<213>  Artificial Sequence

<220>
<223>  VvSAND reverse primer

<400>  10
gtacgactca ctatagggaa cctgaagctt gccttgtcgc                            40


<210>  11
<211>  38
<212>  DNA
<213>  Artificial Sequence

<220>
<223>  VvTIP41 forward primer

<400>  11
aggtgacact atagaatagc taccggaaac cagcgggc                              38


<210>  12
<211>  41
<212>  DNA
<213>  Artificial Sequence

<220>
<223>  VvTIP41 reverse primer

<400>  12

gtacgactca ctatagggag caatccatgc cgtccatccg t 41

<210> 13
<211> 41
<212> DNA
<213> Artificial Sequence

<220>
<223> VvGT14 forward primer

<400> 13
aggtgacact atagaatatg tggcttcatg gcctatgtgc a 41

<210> 14
<211> 42
<212> DNA
<213> Artificial Sequence

<220>
<223> VvGT14 reverse primer

<400> 14
gtacgactca ctatagggac cggaagtagc tgaagaggac ca 42

<210> 15
<211> 41
<212> DNA
<213> Artificial Sequence

<220>
<223> VvGT15 forward primer

<400> 15
aggtgacact atagaatatc cggacggctt atctgatggc c 41

<210> 16
<211> 43
<212> DNA
<213> Artificial Sequence

<220>
<223> VvGT15 reverse primer

<400> 16
gtacgactca ctatagggat gaggggatga agctcaggca ctg 43

<210> 17
<211> 43
<212> DNA
<213> Artificial Sequence

<220>
<223> VvGT16 forward primer

<400> 17
aggtgacact atagaatagc agtttcgccc tcttattgat gga 43

<210>  18
<211>  41
<212>  DNA
<213>  Artificial Sequence

<220>
<223>  VvGT16 reverse primer

<400>  18
gtacgactca ctatagggaa gctttggaag cactgtccgt t                          41


<210>  19
<211>  21
<212>  DNA
<213>  Artificial Sequence

<220>
<223>  VvGT14 forward primer

<400>  19
aatgggttcc atggagaagc c                                                21


<210>  20
<211>  23
<212>  DNA
<213>  Artificial Sequence

<220>
<223>  VvGT14 reverse primer

<400>  20
ggaaagttga gatctagaga agc                                              23


<210>  21
<211>  21
<212>  DNA
<213>  Artificial Sequence

<220>
<223>  VvGT15 forward primer

<400>  21
tcacctctca actcttccac g                                                21


<210>  22
<211>  23
<212>  DNA
<213>  Artificial Sequence

<220>
<223>  VvGT15 reverse primer

<400>  22
gagtttggtt gtacacaact ctg                                              23


<210>  23
<211>  18
<212>  DNA

<213>  Artificial Sequence

<220>
<223>  VvGT16 forward primer

<400>  23
tcctccccag agtgcaag                                                    18


<210>  24
<211>  19
<212>  DNA
<213>  Artificial Sequence

<220>
<223>  VvGT16 reverse primer

<400>  24
tgaccaactc ctataacag                                                   19


10

## Description

The present invention relates to a method for identifying a substrate of a glycosyl transferase of a plant, and to a kit comprising a glycosyl transferase of a plant and an aglycone library from said plant.

Due to their manifold physiological activities, glycosylated natural products are important components of e.g. foodstuffs, pharmaceutical products and cosmetics. Thus, glycosylated diterpenes, steroids and flavonoids are for example used in the food industry as sweeteners (steviosides, glycyrrhizin, neohesperidin, dihydrochalcone) and glycosylated flavonoids as bitterns (neohesperidin), while steroid glycosides and glycosides of antibiotics are employed as medicaments and glycoside extracts as cosmetic products.

In nature, regioselective and enantioselective transfer of sugars is catalyzed by nucleoside diphosphate carbohydrate (such as UDP-glucose) dependent glycosyl transferases (UGTs). Glycosyl transferases of small molecules transfer sugar to a multitude of acceptors, such as hormones and secondary metabolites as well as biotic chemicals and abiotic factors and toxins. In plants, a remarkably large array of different small molecules is glycosylated, including terpenoids, alkaloids, cyanohydrins and glucosinolates as well as flavonoids, isoflavonoids, anthocyanidins, phenylpropanoids and phytohormones. The transfer of a carbohydrate group onto a lipophilic acceptor modifies the chemical characteristics and bioactivity of the acceptor and enables it to access membrane transport systems. *In vitro* studies show that a certain glycosyl transferase protein can often glycosylate several different substrates and that often several different glycosyl transferases are capable of glycosylating a certain substrate.

The glycosyl transferase enzymes are encoded in the different organisms by a large multigenic family and can be identified by a common motif in their primary sequence (Plant Secondary Product Glycosyltransferase (PSPG) box). In the wake of large-scale sequencing of the genomes of different organisms, numerous glycosyl transferase sequences become available, and there is considerable interest in methods for swift identification of their substrate specificity. However, the identification of the physiological substrates of the encoded glycosyl transferase enzymes could not keep pace, even though high throughput methods for the screening of substance databases were developed.

Since enzymes involved in secondary metabolism are rather promiscuous with respect to their substrate specificity, forward and reverse genetic approaches are frequently applied to reveal *in planta* functions of genes. However, in non-model plant these techniques are not yet very well established. Thus, alternative methods are needed.

Typically, identification of glycosyl transferase substrates is carried out by heterologous expression of the glycosyl transferase followed by random high throughput screening of substance databases. Such an approach is often inefficient, tedious, time-intensive and costly.

Thus, there is a need in the art for improved methods and reagents for the identification of the substrates of plant glycosyl transferases. Moreover, there is a need in the art for more efficient, less time-intensive and/or more cost-effective methods and reagents for the identification of the substrates of plant glycosyl transferases.

It is therefore an object of the present invention to provide for improved methods and reagents for the identification of the substrates of plant glycosyl transferases, in particular of UDP-dependent glucosyl transferases. Furthermore, it is an object of the present invention to provide for more efficient, less time-intensive and/or more cost-effective methods and reagents for the identification of glycosyl transferase substrates, in particular of UDP-dependent glucosyl transferases.

These objects are solved by the below-described aspects of the present invention, in particular by a method for identifying a substrate of a glycosyl transferase of a plant according to claim 1 and a kit according to claim 15 comprising separately a glycosyl transferase of a plant and an aglycone library. Preferable embodiments are defined in the dependent claims.

In a first aspect, the present invention relates to a method for identifying a substrate of a glycosyl transferase of a plant, said method comprising the following steps:
a) obtaining a tissue or mixture of tissues from said plant or from a different plant that is phylogenetically closely related to said plant such that said plant and the different plant that is phylogenetically closely related to said plant have overlapping spectra of glycosyl transferase genes and glycosides;
b) obtaining from said tissue or mixture of tissues an extract comprising glycosides, said glycosides consisting of an aglycone component linked to a glycone component;
c) cleaving the glycosides comprised in the extract obtained in step b) to yield free aglycone components not linked to a glycone component, thereby forming an aglycone library;
d) carrying out a reaction of said aglycone components obtained in step c) with one or more activated sugar precursors in the presence of said glycosyl transferase to yield glycosides composed of an aglycone component linked to a glycone component;
e) identifying the glycosides obtained in step d);
wherein the aglycone components of the glycosides identified in step e) are thereby identified as substrates of said glycosyl transferase.

"Glycosyl transferases" are enzymes of EC class 2.4 that catalyze the transfer of a monosaccharide moiety from an activated sugar precursor to an acceptor molecule under formation of a glycosidic linkage (see, e.g., Bowles et al., 2006). The activated sugar precursor serves as glycosyl donor and can be, for example, UDP(uridine diphosphate)-glucose, UDP-xylose, UDP-glucuronic acid, UDP-arabinose, UDP-rhamnose, UDP-galactose, GDP(guanosin diphosphate)-fucose, GDP-mannose or CMP(cytidine monophosphate)-sialic acid. The acceptor molecule may be an alcohol, thiol or acid, preferably an alcohol, such as the alcohol of a terpenoid, alkaloid, cyanohydrin, glucosinolate, flavonoid, isoflavonoid, anthocyanidin, phenylpropanoid, polyphenol, hydroquinone, amine, carbohydrate (monomeric or oligomeric), fatty acid or lipids. Examples of glycosyl transferases are UDP-glucosyltransferases, UDP-arabinosyltransferases, UDP-glucuronosyltransferases, UDP-xylosyltransferases, UDP-galactosyltransferases, UDP-rhamnosyltransferases, GDP-fucosyltransferase, GDP-mannosyltransferase, or CMP-sialyltransferase.

The term "tissue", as used herein, is meant to designate an ensemble of similar cells from the same origin that together carry out a specific function. In some embodiments, the term "tissue" includes several different groups of similar cells. A "mixture of tissues" is a collection comprising more than one tissue, wherein the cells of the different tissues may or may not be intermingled with each other. Obtaining a tissue or mixture of tissues in step a) can be accomplished by cutting off the desired parts of the plant or by dissecting the plant and isolating the desired tissue or tissues.

If the present application refers to a plant A that is "phylogenetically closely related" to a certain plant B or indicates that plant A and plant B are "closely phylogenetically related", this means that individuals of plant A resemble individuals of plant B and are able to breed with individuals of plant B. For example, plants belonging to the same plant family (such as apple, pear and strawberry which all belong to the plant family of Rosaceae) are phylogenetically closely related.

If the present application indicates that two plants "have overlapping spectra of glycosyl transferase genes", this is meant to designate that the two plants are able to express glycosyltransferases from glycosyl transferase genes that form identical glycosides and thus have identical functions.

If the present application indicates that two plants "have overlapping spectra of glycosides", this is meant to designate that the two plants form at least one chemically identical glycoside.

The term plant "family", as used herein, is meant to designate a taxonomic group of plants containing one or more genera which have botanical features in common such as appearance of leaves, stem, roots, flowers and seeds. The names of plant families end in -aceae.

Preferably, said plant (i.e. the plant which the glycosyl transferase the substrate(s) of which are to be identified is derived from) and the plant that is phylogenetically closely related to said plant belong to different families within the same order or to different genera within the same family or to different species within the same genus or to different subspecies, ecotypes or cultivars within the same species or to different isolates or clones of the same variety.

If the present application indicates that two plants "belong to different ecotypes within the same species", this means that the two plants belong to two genetically distinct geographic varieties within the same species.

An "isolate", as used herein, is a population of organisms that is genetically distinct from other organisms of the same species.

In step c), cleaving the glycosides comprised in the extract can be achieved by solvolysis, for example by water (hydrolysis) yielding aglycones and glycones, methanol or ethanol (alcoholysis) yielding aglycones and methylated or ethylated glycones, ammonia (ammonolysis) yielding aglycones and aminoglycosides, or amine (aminolysis) yielding aglycones and aminoglycosides. By cleaving the glycosides comprised in the extract into a mixture comprising free aglycone components not linked to a glycone component and free glycone components not linked to an aglycone component, an aglycone library is obtained. Preferably, in step c) cleaving the glycosides comprised in the extract is carried out by solvolysis, more preferably by thiolysis (i.e. cleaving the glycoside into the glycone and the aglycone component through a reaction with a thiol (R-SH)) or hydrolysis, more preferably by hydrolysis.

In one embodiment, in step a) a mixture of tissues from a plant comprises all tissues of said plant, i.e. the whole plant with all its tissues is used.

To obtain an extract from the tissue or mixture of tissues, the tissue or mixture of tissues can be homogenized, for example by freezing it in liquid nitrogen and mechanically grinding it. Subsequently, the homogenate can be extracted with a solvent, for example water, methanol, acetonitrile, 0.1 M phosphate buffer, 13% ethanol. The skilled person is aware how by choosing appropriate solvents and extraction conditions specific substances can be extracted. For example, XAD solid phase extraction is used to selectively enrich glycosides with molecular weights less than 3000 Da, separate glycosides that contain proteins as aglycones, and to concentrate terpene and aromatic glycosides. Glycosides of macromolecules, proteins and polysaccharides are removed by elution with water whereas glycosides of small molecules are eluted by polar organic solvents, for example methanol, acetonitrile, ethyl acetate or acetone. Alternatively, RP18 solid phase extraction is applied.

A "glycoside", as used herein, is a molecule in which a sugar is bonded to a non-sugar via a glycosidic bond. The sugar group forms the "glycone" part or "glycone component" of the glycoside, the non-sugar group the "aglycone" part or "aglycone component". Accordingly, a glycoside may consist of a sugar as glycone component (designated "Z" in the general chemical structure below) linked through its anomeric carbon atom to the hydroxy group of an alcohol (chemical structure R-OH) as aglycone component, thus resulting in a glycoside of the general chemical structure R-O-Z. For example, in the glycoside linaloyl β-D-glucoside, the glycone component glucose is linked to the aglycone component linalool.

In some embodiments the aglycone component of the glycoside can be a carbonic acid (R-COOH), resulting in a glycoside of the general chemical structure R-C(O)O-Z. In other embodiments, the aglycone component of the glycoside is not a carbonic acid. In some embodiments the aglycone component of the glycoside can be a thiol (R-SH), resulting in a glycoside of the general chemical structure R-S-Z. In other embodiments, the aglycone component of the glycoside is not a thiol. In some embodiments, the term "glycoside" does not include glycosylic amines (R-NR'-Z).

The glycone component can be, for example, glucose, xylose, galactose, glucuronic acid, arabinose, rhamnose, galactose, fucose, mannose or sialic acid. Preferably, the glycone component is glucose.

To carry out the reaction of step c), the aglycone components obtained in step c) are mixed with one or more activated sugar precursors (such as UDP-glucose, UDP-xylose, UDP-glucuronic acid, UDP-arabinose, UDP-rhamnose, UDP-galactose, GDP-fucose, GDP-mannose or CMP-sialic acid), as well as the glycosyl transferase the substrates of which are to be identified. For example, a mixture containing 100 µL purified enzyme (50 µg), 100-150 µL Tris-HCl buffer (100 mM, pH 7.5, 10 mM 2-mercaptoethanol), 37 pmol UDP-glucose and 50-100 µL extract (10 mg extract dissolved in 1 mL of methyl-tert-butylether) are incubated at 30°C for 24 hr. This results in the formation of glycosides composed of an aglycone component linked to a glycone component.

Subsequently, in step e) the glycosides obtained in step d) are identified among the aglycones of the aglycone library that did not form glycosides during the reaction of step d). This identification may for example involve LC-MS/MS (liquid chromatography-tandem mass spectrometry), GC-MS (gas chromatography-mass spectrometry), FTIR (Fourier Transformation Infra-red Resonance) spectrometry, NMR (Nuclear Magnetic Resonance) spectroscopy, HPLC (High Performance Liquid Chromatography) or TLC (Thin Layer Chromatography). The aglycone components of the glycosides identified in step e) are thereby identified as substrates of the glycosyl transferase.

In some embodiments, the plant tissue or mixture of plant tissues obtained in step a) shows expression of the mRNA coding for said glycosyl transferase, preferably as detected by real-time PCR analysis or by next generation sequencing of mRNA, preferably RNAseq, and/or glycosyl transferase protein is detected in said plant tissue or mixture of plant tissues, preferably by Western blotting.

If the present application refers to a tissue that "shows expression" of a certain mRNA, this means that in an assay that allows detection of a specific mRNA, such as real-time PCR (quantitative PCR) analysis, next generation sequencing of mRNA, preferably RNAseq, in situ hybridization or Northern blotting, a positive signal above background is detected with said tissue. If the present application specifies that "glycosyl transferase protein is detected" in a certain plant tissue, this means that in an assay that allows specific detection of a certain protein, such as Western blotting with a specific antibody, a positive signal above background is detected with said tissue.

"Next-generation sequencing" refers to high-throughput sequencing technologies that parallelize the sequencing process, producing thousands or millions of sequences concurrently. RNAseq refers to RNA sequencing of the whole transcriptome of e.g. a tissue, for example by using randomly primed cDNA and massively parallel short-read sequencing.

In some embodiments, the plant tissue or mixture of plant tissues obtained in step a) shows a higher expression of the mRNA coding for said glycosyl transferase than other tissues of the same plant, preferably as detected by real-time PCR analysis or by next generation sequencing of mRNA, preferably RNAseq, and/or a higher protein level of said glycosyl transferase than other tissues of the same plant, preferably as detected by comparative protein analysis by MALDI-TOF mass spectrometry.

At some instances, the present application refers to a tissue showing "a higher expression of a certain mRNA than other tissues of the same plant". This means that in an assay that allows for quantitative or semi-quantitative detection of a specific mRNA, such as real-time PCR (quantitative PCR) analysis, next generation sequencing of mRNA, preferably RNAseq, in situ hybridization or Northern blotting, a stronger signal is obtained with that tissue compared to other tissues of the same plant. If the present application refers to a tissue showing "a higher protein level of said glycosyl transferase than other tissues of the same plant", this means that in an assay that allows for comparison of the protein level of said glycosyl transferase in different tissues, such as comparative protein analysis by MALDI-TOF mass spectrometry, a stronger signal is obtained with that tissue compared to other tissues of the same plant.

In some embodiments, the plant tissue or mixture of plant tissues obtained in step a) is from a developmental stage of the plant from which the plant tissue or mixture of plant tissues is obtained in which said plant tissue shows in real-time PCR analysis a higher expression of the mRNA coding for said glycosyl transferase than in other developmental stages of the same plant, and/or shows in comparative protein analysis by MALDI-TOF mass spectrometry a higher protein level of said glycosyl transferase than in other developmental stages of the same plant.

At some instances, the present application refers to a tissue obtained from a developmental stage of a plant "in which said plant tissue shows in real-time PCR analysis a higher expression [of a certain mRNA] than in other developmental stages of the same plant". This means that, if a certain tissue is obtained from a plant at a certain developmental stage (such as during sprout development, vegetative growth or flowering) and analysis by real-time PCR (quantitative PCR) is carried out, the signal detected with the tissue from said developmental stage is stronger than compared to the same type of tissue obtained from a different developmental stage of the same plant.

In some embodiments, prior to step a), the plant from which the tissue or mixture of tissues is obtained in step a) is exposed to an environmental stimulus, preferably an environmental stimulus selected from the group consisting of exposure to visible and/or ultra-violet light, a less than ideal supply of water or nutrients, exposure to a chemical, exposure to a pathogen or symbiont, higher or lower than standard atmospheric pressure (101325 Pa), exposure to an altered atmospheric composition compared to standard atmospheric composition, exposure to a putative glycosyl transferase substrate or one of its precursors, exposure to an enzyme inhibitor which changes substrate flow, a temperature that is higher or lower than the temperature at which the plant is commonly grown, mechanical stress, exposure to a phytohormone, preferably a phytohormone involved in pathogen or stress signaling, more preferably a phytohormone selected from the group consisting of jasmonic acid, salicylic acid, nitrogen monoxide, ethylene and a systemic peptide, exposure to a transgenic or transiently expressed regulatory protein, and exposure to a silencing RNA, wherein, preferably, said environmental stimulus results in an increase in the expression of the mRNA coding for said glycosyl transferase in said plant tissue, preferably as detected by real-time PCR analysis or by next generation sequencing of mRNA, preferably RNAseq, and/or in an increase in the protein level of said glycosyl transferase, preferably as detected by comparative protein analysis by MALDI-TOF mass spectrometry.

Exposure to visible and/or ultra-violet light can be achieved by shining visible and/or ultra-violet light, preferably of a defined intensity, at the plant.

A less than ideal supply of water or nutrients can be identified by testing different supplies of water or nutrients and detecting under which conditions the highest rate of plant growth is observed. A less than ideal supply of water is a water supply below the water supply that results in the highest rate of plant growth. A less than ideal supply of nutrients is a supply of nutrients below the supply of nutrients that results in the highest rate of plant growth.

Exposure to a chemical may for example be exposure to environmental chemicals or toxins known to provoke strong responses in plants like aromatic compounds, salts of heavy metals, or plants toxins like juglone. This can be achieved by including the chemical in the water supply used for irrigating the plant.

Exposure to a pathogen or symbiont may for example be exposure to general plant pathogens specific for this species, or related species (mostly bacteria, fungi), e.g. fire blight: *Erwinia amylovora* for *Malus* spp. and *Pyrus* spp. (bacterium), apple scab: Venturia inaequalis for *Malus* spp. (fungus), *Botrytis cinerea* (fungus) for grape *Vitis vinifera* and strawberry *Fragaria vesca* and *Fragaria x ananassa.* Symbionts are e.g. mycorrhizal fungi and e.g. *Glomus* spp. This can be achieved by including the chemical or pathogen in the water supply used for irrigating the plant or by syringe injection of the chemical or pathogen into the tissue or by grafting with an pathogen infected scion.

Exposure to altered atmospheric composition compared to standard atmospheric composition can be achieved by growing the plant in an atmosphere that contains a concentration of gases that differs from the standard concentrations, such as in an atmosphere containing CO₂ at an increased concentration compared to the standard CO₂ concentration of about 0.04 % by volume.

Exposure to a putative glycosyl transferase substrate or one of its precursors can for example be exposure to cinnamic acid to induce expression of cinnamic acid glycosyltransferase or to chalcone for induction of flavonoid glycosyltransferases .This can be achieved by including the substrate or one of its precursors in the water supply used for irrigating the plant or by syringe injection of the substrate or one of its precursors into the tissue.

Exposure to an enzyme inhibitor which changes substrate flow can for example be exposure to the 2-oxoglutarate analogon Prohexadione-Ca in a concentration of 250 ppm to induce flavonoid enzymes in apple *(Malus* spp.) which include 2-oxoglutarate dependent flavonoid enzymes. Exposure to such an enzyme inhibitor may for example be achieved by including the inhibitor in the water supply used for irrigating the plant or by syringe injection of the inhibitor into the tissue.

A temperature that is higher or lower than the temperature at which the plant is commonly grown as environmental stimulus means that the plant is grown at a temperature that is higher or lower than the temperature at which the plant is usually grown. For a plant that is usually grown at room temperature (25° C), growth at a higher temperature could for example be growth at 35° C, and growth at a lower temperature could for example be growth at 10° C (+/-15°C based on plant usual temperature). If there is no temperature at which the plant is usually grown, then higher/lower temperature means a temperature that is higher/lower than the temperature at which the plant shows highest growth rate during development.

Mechanical stress as environmental stimulus can for example be applied by wind simulation, bending of leaves or loading with excessive weight.

Phytohormones involved in pathogen or stress signaling are for example jasmonic acid, salicylic acid or ethylene. Examples for systemic peptides are systemin and AtPEPs. Exposure to a phytohormone or a systemic peptide can be achieved by including the phytohormones or systemic peptides in the water supply used for irrigating the plant or by syringe injection of the phytohormones or systemic peptides into the tissue.

Exposure to a transgenic or transiently expressed regulatory protein can for example be achieved by agroinfiltration of a transcription factor gene (leaf colour gene from *Zea mays, Lc* gene) driven by a 35S promotor.

A silencing RNA can for example be administered to the plant by virus induced gene silencing (VIGS).

Whether an environmental stimulus is an environmental stimulus that results in an increase in the expression of the mRNA coding for said glycosyl transferase in said plant tissue can be verified by quantifying, for example by real-time PCR analysis, the amount of the mRNA coding for said glycosyl transferase present in the plant tissue obtained from a plant that has been exposed to said stimulus and the amount of said mRNA present in the plant tissue obtained from a plant that has not been exposed to said stimulus. If the amount of said mRNA detected in the plant tissue obtained from the plant that has been exposed to said stimulus is higher than in the plant tissue obtained from the plant that has not been exposed to said stimulus, the environmental stimulus is an environmental stimulus that results in an increase in the expression of the mRNA coding for said glycosyl transferase in said plant tissue. Whether an environmental stimulus is an environmental stimulus that results in an increase in the protein level of said glycosyl transferase in said plant tissue can be verified for example by carrying out a comparative protein analysis by MALDI-TOF mass spectrometry, comparing the amount of said glycosyl transferase protein in the plant tissue obtained from a plant that has been exposed to said stimulus with the amount of said glycosyl transferase protein in the plant tissue obtained from a plant that has not been exposed to said stimulus. If the amount of said glycosyl transferase protein detected in the plant tissue obtained from the plant that has been exposed to said stimulus is higher than in the plant tissue obtained from the plant that has not been exposed to said stimulus, then it can be concluded that the environmental stimulus is an environmental stimulus that results in an increase in the protein level of said glycosyl transferase in said plant tissue.

In some embodiments, in step b) said aglycone component of said glycosides is an aliphathic or aromatic alcohol, thiol, amine or acid, preferably an aliphathic or aromatic alcohol.

In some embodiments, in step b) said glycosides have a molecular weight of below 3 000 Dalton, preferably below 1 700 Dalton. Whether said glycosides have a molecular weight of below 3 000 Dalton or below 1 700 Dalton can be verified by LC-MS analysis.

In some embodiments, in step b) said aglycone component of said glycosides is not a protein. Whether the aglycone component of said glycosides is not a protein can be verified by Bradford test, Coomassie- or silver staining after SDS-PAGE analysis.

In some embodiments, in step b) said glycosides are terpene glycosides, preferably mono-, sesqui- and/or diterpene glycosides, more preferably monoterpene glycosides. Whether said glycosides are terpene glycosides or mono-, sesqui- and diterpene glycosides or enriched monoterpene glycosides can be verified by LC-MS (liquid chromatography-tandem mass spectrometry), FTIR (Fourier Transformation Infra-red Resonance) or NMR (Nuclear Magnetic Resonance) analysis.

The term "terpene glycoside" refers to a glycoside the aglycone component of which is a terpenoid. In some embodiments, the term does not include glycosides the aglycone component of which is a terpenoid in which methyl groups have been moved or removed compared to a terpene formally derived from isoprene units. In some embodiments, the term does not include glycosides the aglycone component of which includes further functional groups in addition to the functional group forming the linkage to the glycone component of the terpene glycoside. In some embodiments, the term does not include glycosides the aglycone component of which includes further oxygen atoms in addition to oxygen atom(s) that are part of the functional group forming the linkage to the glycone component of the terpene glycoside. The term "monoterpene glycoside" refers to a glycoside the aglycone component of which is a monoterpenoid (formally comprising two isoprene units, such as geraniol, citronellol or linalool). The term "sesquiterpene glycoside" refers to a glycoside the aglycone component of which is a terpenoid formally comprising three isoprene units (such as farnesol). The term "diterpene glycoside" refers to a glycoside the aglycone component of which is a diterpenoid (formally comprising four isoprene units, such as steviol).

In some embodiments, in step b) said aglycone component of said glycosides is an aromatic compound, preferably phenethyl alcohol, raspberry ketone and/or furaneol.

In some embodiments, the extract obtained in step b) does not comprise the glycosides of macromolecules, proteins and/or carbohydrates present in said plant tissue or mixture of plant tissues. Whether the extract does not comprise such glycosides can be verified by size exclusion chromatography and MALDI-TOF (Matrix Assisted Laser Desorption Ionization Time-of-Flight) mass spectrometry.

In some embodiments, obtaining said extract in step b) involves extraction of the tissue followed by solid phase absorption and consecutive elution with a nonpolar organic solvent, preferably with diethyl ether or dichloromethane, followed by elution with a polar organic solvent, preferably with methanol or acetonitrile or ethyl acetate, to separate free monoterpenes from monoterpene glycosides. The nonpolar organic solvent is used to elute free monoterpenes whereas the polar organic solvent is utilized to extract monoterpene glycosides.

In some embodiments, in step d) a glycosyl transferase is used which is
- a recombinantly expressed glycosyl transferase or
- a preparation of said glycosyl transferase obtained by purification or partial purification from said plant (i.e. the plant which the glycosyl transferase the substrate(s) of which are to be identified is derived from), preferably from said tissue or mixture of tissues of said plant (i.e. the plant which the glycosyl transferase the substrate(s) of which are to be identified is derived from).

A recombinantly expressed glycosyl transferase is a glycosyl transferase protein that has been expressed from a recombinant DNA molecule, i.e. from a DNA molecule formed by laboratory methods of genetic engineering (such as molecular cloning) to bring together genetic material from multiple sources, creating a DNA sequence that would not be found naturally in a biological organism. Typically, a recombinantly expressed glycosyl transferase is expressed by heterologous expression (i.e. in a host organism which is different from the organism from which said glycosyl transferase is originally derived), such as by expression in e.g. *E. coli, Saccharomyces cerevisiae, Pichia pastoris* or insect cells, preferably in *E. coli.* Preferably, said recombinantly expressed glycosyl transferase is expressed by heterologous expression. Preferably, said recombinantly expressed glycosyl transferase is isolated after expression from other proteins of the host organism by methods of protein purification.

A preparation of said glycosyl transferase obtained by purification or partial purification from said plant can be provided by common methods of protein purification known to a person of skill in the art, for example a purification protocol involving methods such as mechanical homogenization of a plant tissue, solvent extraction of proteins, differential centrifugation and/or steps of column chromatography (e.g. ion-exchange chromatography or gel filtration chromatography). Preferably, said glycosyl transferase is not a recombinantly expressed glycosyl transferase. Preferably, said preparation of said glycosyl transferase obtained by purification is a (substantially) homogeneous preparation of said glycosyl transferase. A preparation of said glycosyl transferase obtained by partial purification refers to a situation where the purification has resulted in enrichment for the glycosyl transferase compared to the tissue used as starting point for the partial purification, but where other proteins besides said glycosyl transferase are still present after the partial purification.

In some embodiments, said activated sugar precursors used in step d) are selected from the group consisting of UDP-glucose, UDP-xylose, UDP-galactose, UDP-glucuronic acid, UDP-arabinose and UDP-rhamnose, wherein, preferably, said activated sugar precursor is UDP-glucose.

In some embodiments, said identification in step e) is carried out by LC-MS/MS (liquid chromatography-tandem mass spectrometry), GC-MS (gas chromatography-mass spectrometry), FTIR (Fourier Transformation Infra-red Resonance) spectrometry, NMR (Nuclear Magnetic Resonance) spectroscopy, HPLC (High Performance Liquid Chromatography) or TLC (Thin Layer Chromatography).

In some embodiments, the plant from which the tissue or mixture of tissues is obtained in step a) has a content of terpene glycosides of at least 0.01 milligrams per gram of dry weight of the plant. To determine the content of terpene glycosides in a plant, material of the plant is dried and weighed; from an equivalent amount of material of the plant terpene glycosides are purified by a purification protocol involving the steps of solvent extraction, solid phase extraction, and reversed phase ornormal phase or counter current chromatography; the isolated terpene glycosides are dried and weighed or alternatively quantified by LC-MS analysis; from the ratio of the dry weight of the isolated terpene glycosides to dry weight of the total plant material the content of terpene glycosides can be determined.

In some embodiments, said cleaving of step c) is carried out by enzymatic hydrolysis with a hydrolase or mixture of hydrolases, wherein, preferably, said hydrolase or mixture of hydrolases is selected from the group consisting of a glycosidase, a mixture of glycosidases, an esterase, a mixture of esterases, a mixture of one or more glycosidases with one or more esterases, a glucosidase and a mixture of glucosidases, wherein, more preferably said hydrolase or mixture of hydrolases is a glucosidase or a mixture of glucosidases, to yield an aglycone library prepared by enzymatic hydrolysis,
or wherein said cleaving of step c) is carried out by hydrolysis with an acid, preferably with phosphoric acid, hydrochloric acid, trichloroacetic or sulphuric acid, to yield an aglycone library prepared by hydrolysis with an acid.

Esterases allow for cleaving of glycosides having an aglycone component which is a carbonic acid (R-COOH), i.e. glycosides of the general chemical structure R-C(O)O-Z.

In some embodiments, after step c) and prior to step d), one or more of the following is done:
- said hydrolase or mixture of hydrolases is inactivated, preferably by thermic inactivation, to yield an aglycone library prepared by enzymatic hydrolysis with inactivated hydro lase(s);
- an inhibitor of said hydrolase(s) is added, to yield an aglycone library prepared by enzymatic hydrolysis including hydrolase/glycosidase/glucosidase inhibitor;
- the free aglycone components obtained in step c) are separated from said hydrolase(s) to yield a purified aglycone library prepared by enzymatic hydrolysis.

In some embodiments, after step c) and prior to step d), the free aglycone components obtained in step c) are isolated from the other reaction components present during the cleaving of step c), to yield a purified aglycone library.

Separation of the free aglycone components obtained in step c) from hydrolase(s) or isolation of the free aglycone components obtained in step c) from the other reaction components present during the cleaving of step c) can be accomplished by common methods for the purification of chemical compounds and separation of proteins known the person of skill in the art, including methods like extraction with solvents, salting out proteins, ultrafiltration, column chromatography, and/or solid phase extraction.

In a second aspect, the present invention relates to a kit comprising, preferably separately,
A) a glycosyl transferase of a plant, wherein, preferably, said glycosyl transferase is a recombinantly expressed glycosyl transferase; and
B) an aglycone library as defined in any of the embodiments above.

Preferably, said aglycone library of B) is an aglycone library prepared from a tissue or mixture of tissues of the plant which the glycosyl transferase of A) is derived from or of a plant that is phylogenetically closely related to the plant which the glycosyl transferase of A) is derived from. Preferably, the plant which the glycosyl transferase of A) is derived from and the plant that is phylogenetically closely related to said plant have overlapping spectra of glycosyl transferase genes and glycosides. Preferably, the plant which the glycosyl transferase of A) is derived from and the plant related to said plant belong to different families within the same order or to different genera within the same family or to different species within the same genus or to different subspecies, ecotypes or cultivars within the same species or to different isolates or clones of the same variety.

"Instructions for use" of said aglycone library are instructions that provide information how said aglycone library can be used to identify a substrate of a glycosyl transferase of a plant, preferably a substrate of the glycosyl transferase of A).

In some embodiments, said kit further comprises, preferably separately,
D) a mixture of hydrolases showing enzymatic activity to a wide range of glycosides. Examples for suitable mixtures of hydrolases showing enzymatic activity to a wide range of glycosides include, for example, Emulsin (a mixture of enzymes with glucoside hydrolyzing activity extracted from bitter almonds), AR 2000 (an enzyme preparation with glucosidase activity and promiscuous hydrolase activity prepared from *Aspergillus niger;* DSM Food Specialties Beverage Ingredients, Delft, Netherlands), and Rohapect preparations (mixture of hydrolases for pectin and starch degradation from AB Enzymes, Darmstadt).

In a third aspect, the present invention relates to a kit comprising separately
A) accessory material to prepare an aglycone library as defined in any of the embodiments above; and
B) instructions for the preparation of such an aglycone library (i.e. an aglycone library according to any of the embodiments above).

Examples for accessory material to prepare an aglycone library are XAD columns, RP columns or suitable solvents.

"Instructions for the preparation" of an aglycone library are instructions that provide information how said aglycone library can be prepared by the provided material and, preferably, used to identify a substrate of a glycosyl transferase of a plant, preferably a substrate of the glycosyl transferase of A).

In some embodiments, said kit further comprises separately
C) a mixture of hydrolases showing enzymatic activity to a wide range of glycosides.

In some embodiments, said kit further comprises separately
D) a glycosyl transferase of a plant, wherein, preferably, said glycosyl transferase is a recombinantly expressed glycosyl transferase.

The glycosyl transferase can be used as positive control.

Preferably, said kit is for the preparation of an aglycone library as defined in any of the embodiments above.

In the following, reference is made to the figures:

All methods mentioned in the figure descriptions below were carried out as described in detail in the examples.

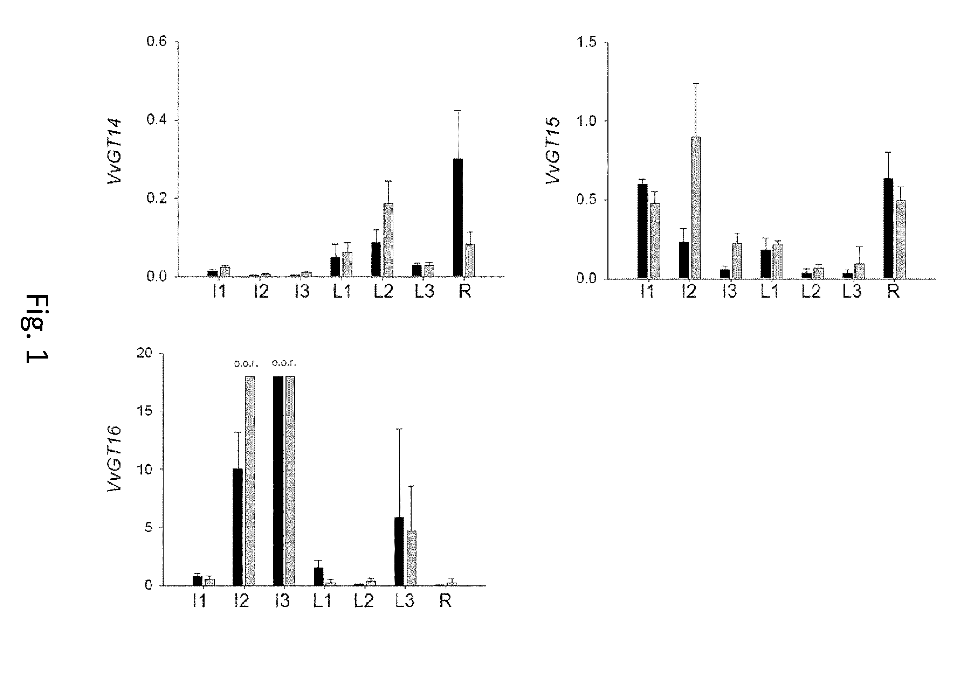

Figure 1 shows experimental data from a gene expression analysis of *VvGTs* by GeXP (Genome Lab GeXP Genetic Analysis System to quantify transcript abundance, see below) in non-berry tissues.

The relative expression was quantified in Gewurztraminer 11-18 Gm (black bars) and White Riesling 239-34 Gm (grey bars). Sampled tissues: Inflorescences four weeks (I1) and two weeks (12) before flowering and at full bloom (I3), leaves at the age of approximately one week (L1), three weeks (L2) and five weeks (L3) and roots (R). The mean values +SD of three independent experiments are shown, o.o.r. out of range.

Figure 2 shows experimental data from a gene expression analysis of *VvGTs* by GeXP. Different stages of berry development are given as weeks post flowering. Expression was determined in berry skins (exocarp) of five different varieties and clones. Mean values ± SD of three independent experiments are shown, o.o.r. out of range.

Figure 3 shows data on the relative specific activity (%) of VvGT14a (A), VvGT15a-c (B), and VvGT16 (C) protein from *Vitis vinifera* towards putative substrates as determined by radiochemical analysis with UDP-[¹⁴C]glucose (see Example 1 below).

The relative activities refer to the highest level of extractable radioactivity which was measured for the conversion of geraniol (100 %) in case of VvGT14a and VvGT15a-c (order of the columns 15c, 15b, 15a) and benzyl alcohol (100 %) in the case of VvGT16. Data for two biological and two technical replicates are shown. Black and white bars represent monoterpenoids and non-monoterpenoids, respectively.

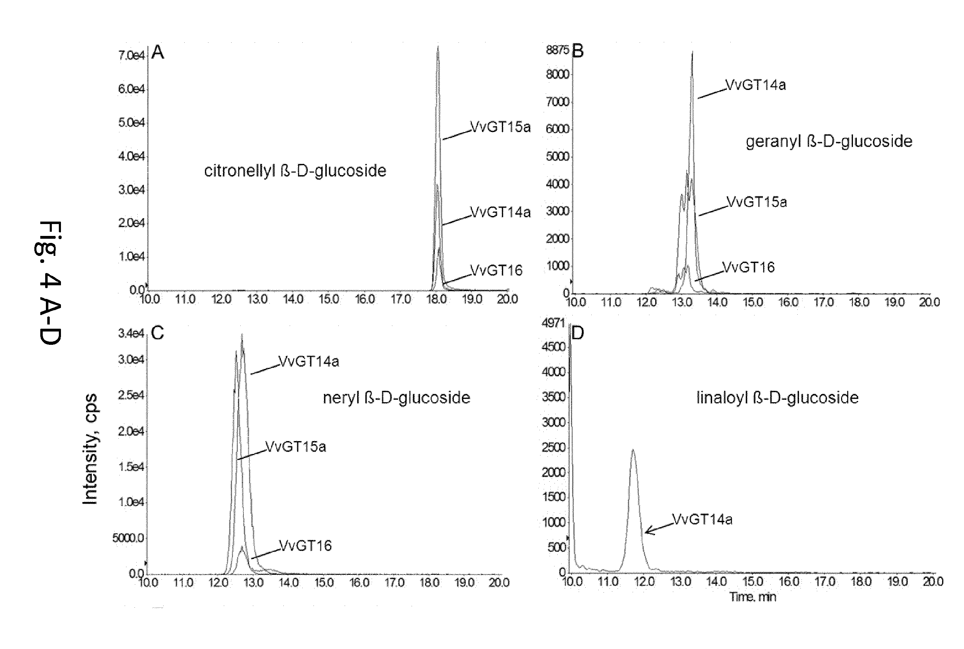

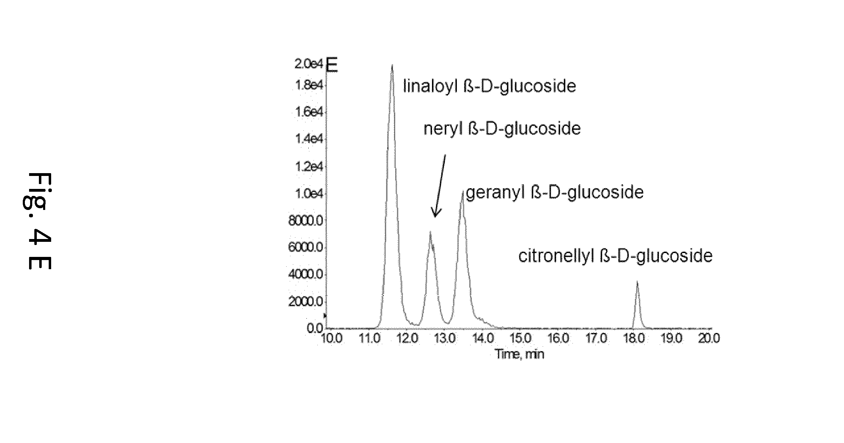

Figure 4 shows the detection of monoterpenyl ß-D-glucosides as products of VvGT14a, VvGT15a and VvGT16 by LC-MS.

LC-MS analysis of citronellyl ß-D-glucoside (A), geranyl ß-D-glucoside (B), neryl ß-D-glucoside (C), linaloyl ß-D-glucoside (D) formed by VvGT14a, VvGT15a and VvGT16 and a mixture of synthesized monoterpenyl ß-D-glucosides (E); chromatograms display an overlay of single product measurements, traces show the total ion current of the characteristic transitions (refer to Example 1), Gaussian smoothing was partly applied.

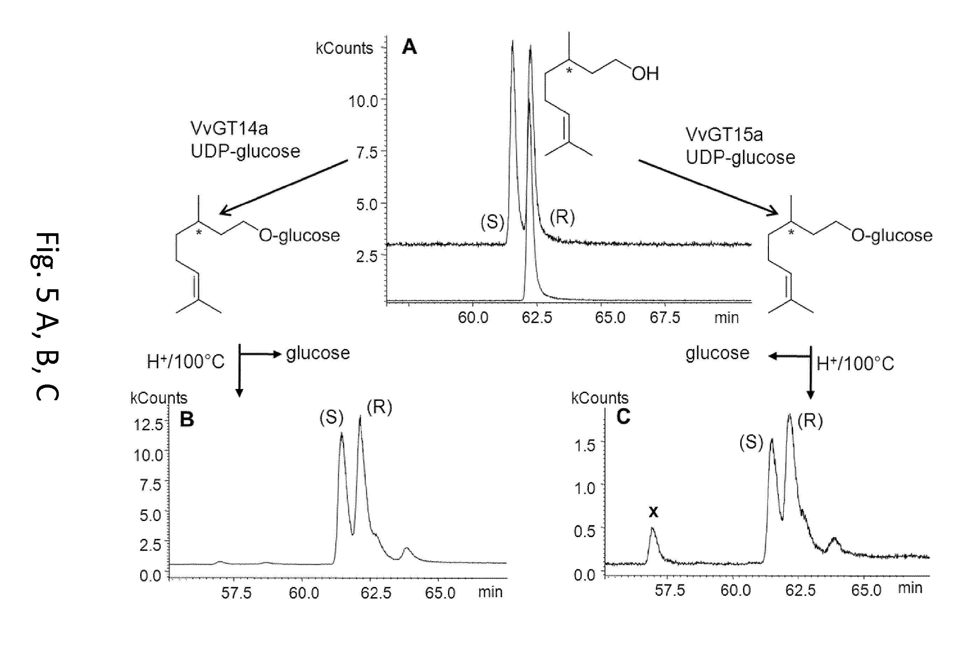

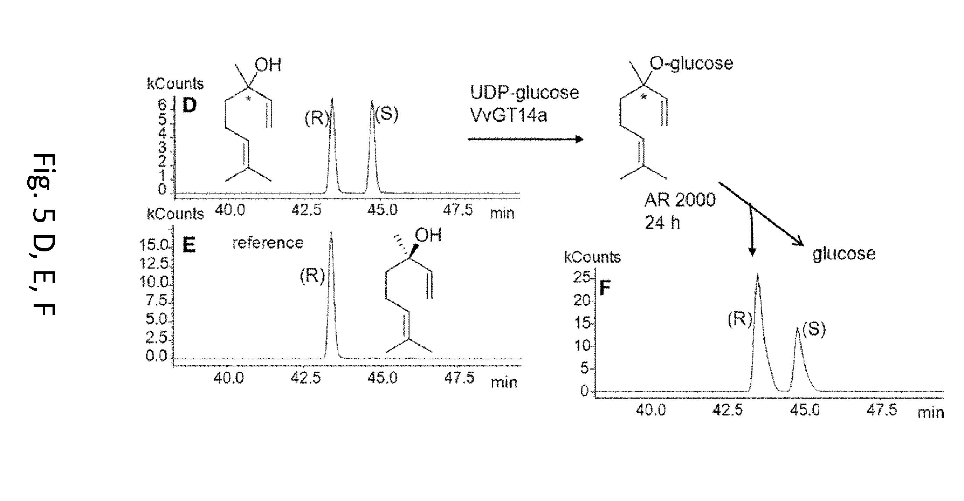

Figure 5 shows experimental data on the enantioselectivity of VvGT14a and VvGT15a as determined by chiral phase SPME-GC-MS analysis of citronellol and linalool. A) Racemic mixture of R,S-citronellol was used as substrate for VvGT14a and VvGT15a, enantiomerically pure R-citronellol was used as reference.

B, C) racemic citronellol is released by acid catalyzed hydrolysis of citronellyl-ß-D-glucoside formed by VvGT14a and VvGT15a. Signals labeled with "*x*" are hydrolysis by-products. Chromatograms are shown in SIM mode by using the characteristic ion traces *m*/*z* 69, 81 and 123 for citronellol.
D) Racemic mixture of *R,S*-linalool was used as substrate for VvGT14a.
E) Enantiomerically pure *R*- linalool was used as reference material.
F) A slight preference for the *R*-linalool is revealed after enzymatic hydrolysis with AR 2000. Chromatograms are shown in SIM mode *(m*/*z* 71, 93).

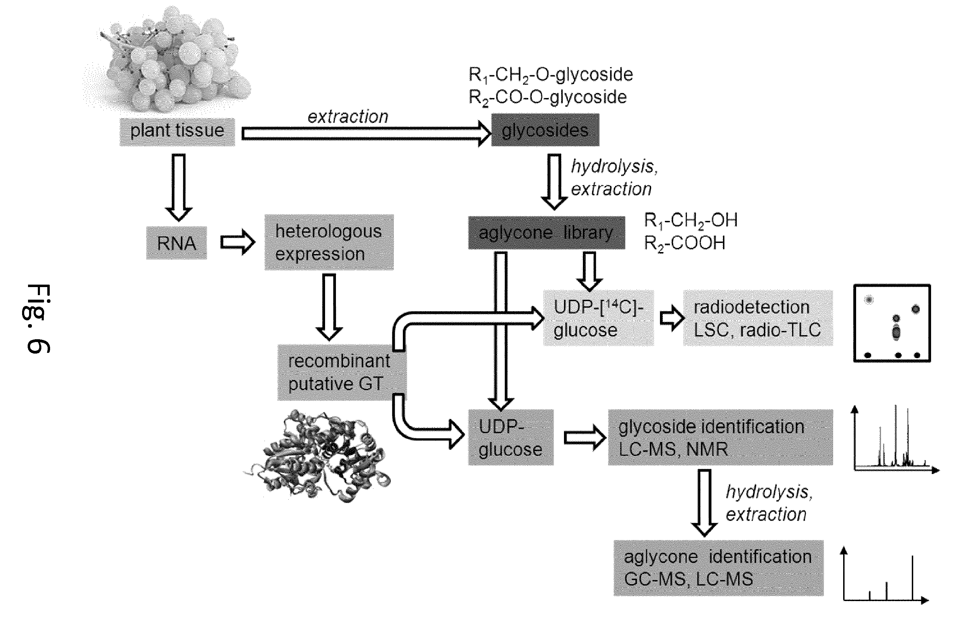

Figure 6 shows a flow chart of the formation and use of a physiologic aglycone library. The approach has several advantages: An aglycone library can be prepared from any tissue that shows high *UGT* transcript levels. The library contains the natural substrates in high concentration as they are enriched during the preparation of the extract and can be easily screened with recombinant UGT enzymes and various labeled and unlabeled UDP-sugars. Formation of products can be rapidly quantified by LSC and confirmed by TLC. Identification of glycosides by LC-MS and NMR analyses is greatly facilitated as levels of impurities are very low. Finally, the structure of the carbohydrate moiety is already known from the used UDP-sugar.

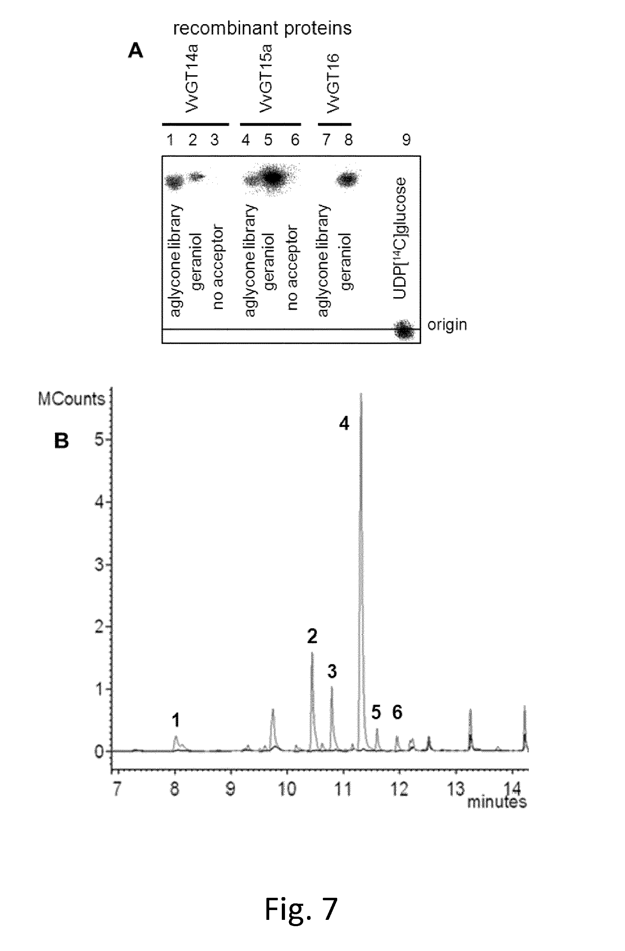

Figure 7 shows experimental data obtained from functional screening of VvGT14a, 15a and 16 by radio-TLC and GC-MS analysis.

Radio-TLC analysis (A) of products formed by VvGT14a (1,2,3), VvGT15a (4,5,6) and VvGT16 (7,8,9) after incubation with the aglycone library obtained from grape berries of Gewurztraminer 11-18 Gm (1,4,7); Positive control: geraniol (2,5,8); negative control: no acceptor molecule (3,6); UDP [¹⁴C]-glucose (9; approximately: 3000 dpm). The plates were analyzed by digital autoradiograph. The products formed from citronellol, geraniol and nerol were verified by LC-MS analysis. GC-MS analysis (B, total ion chromatogram) of volatiles that were enzymatically released from glucosides which were formed by incubation of an aglycone library obtained from grape berries of Gewurztraminer 11-18 Gm with UDP-gucose and VvGT14 (gray) or heat-inactivated VvGT14a (black) as control 1: linalool; 2: citronellol; 3: nerol; 4: geraniol; 5: benzyl alcohol; 6: phenylethanol.

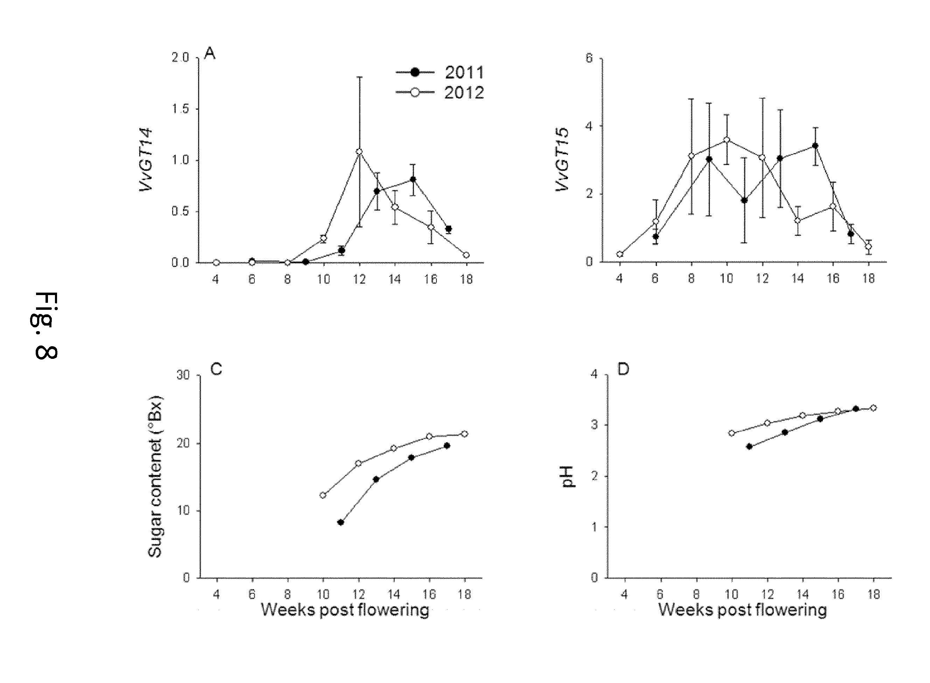

Figure 8 shows experimental data from a gene expression analysis of *VvGTs* by GeXP. Gene expression of *VvGTs* by GeXP (A) was analyzed in berry skins (exocarp) of Muscat FR 90 from two consecutive years (2011, 2012). Mean values ± SD of three independent experiments are shown. The ripening related parameters sugar content (C) and pH (D) were quantified after veraison in must obtained from 100 berries of Muscat FR 90 by refractometer and pH meter.

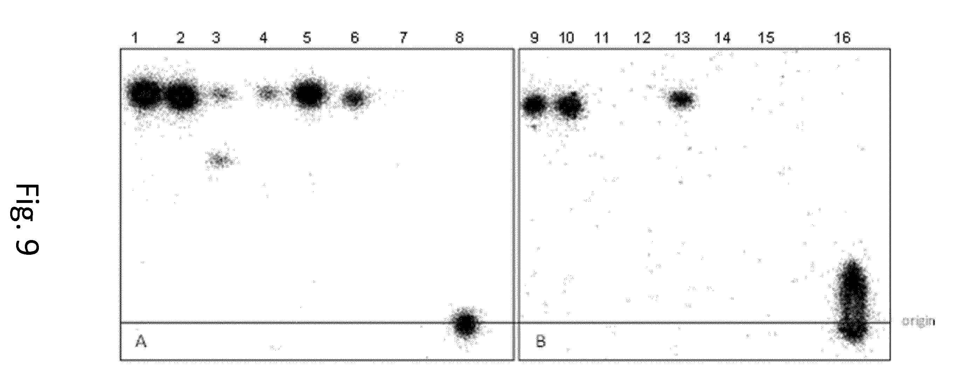

Figure 9 shows experimental data from radio-TLC analysis of products formed by (A) VvGT14a and (B) VvGT16 from different monoterpenes. Citronellol (1,9), geraniol (2,10), 8-hydroxylinalool (3,11), linalool (4,12), nerol (5,13), terpineol (6,14), no substrate (7,15), UDP [¹⁴C]-glucose (8,16, approximately: 3000 dpm). The plates were analyzed by digital autoradiograph. The putative glucosidic products formed from citronellol, geraniol and nerol were verified by LC-MS analysis.

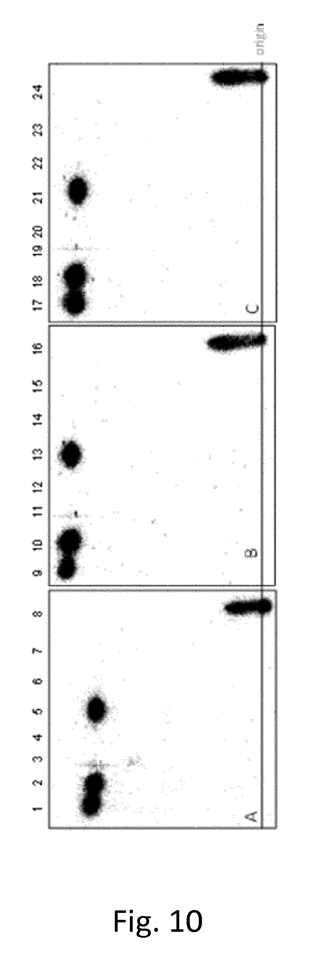

Figure 10 shows experimental data from radio-TLC analysis of products formed by (A) VvGT15a, (B) VvGT15b and (C) VvGT15c from different monoterpenes. Citronellol (1,9,17), geraniol (2,10,18), 8-hydroxylinalool (3,11,19), linalool (4,12,20), nerol (5,13,21), terpineol (6,14,22), no substrate (7,15,23), UDP [¹⁴C]-glucose (8,16,24; approximately: 3000 dpm). The plates were analyzed by digital autoradiograph. The putative glucosidic products formed from citronellol, geraniol and nerol were verified by LC-MS analysis.

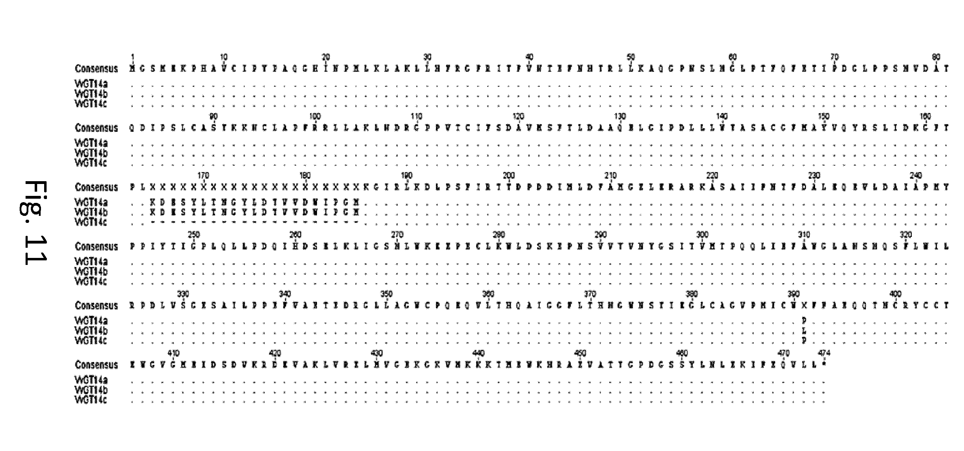

Figure 11 shows a multiple protein sequence alignment of the three variants of VvGT14. The alignment was performed using MUSCLE alignment in Geneious Pro 5.5.6 software (Biomatters). VvGT14a was catalytically active while VvGT14b and VvGT14c did not show any enzymatic activity.

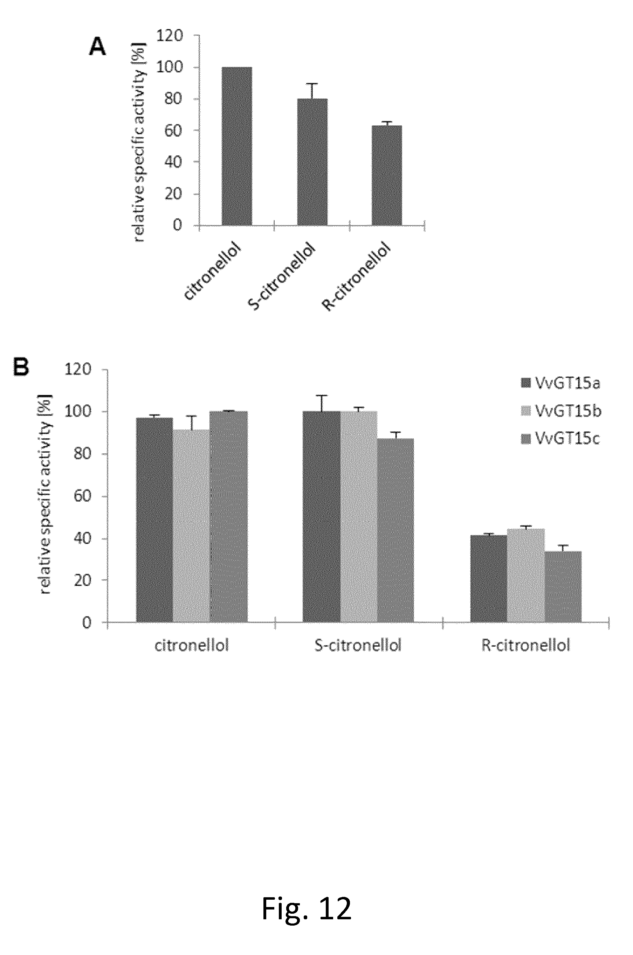

Figure 12 shows experimental data on the enantiomeric discrimination of VvGT enzymes. Relative specific activity (%) of VvGT14a protein from *Vitis vinifera* towards citronellol and enantiomerically pure *R*- and *S*-citronellol (A). The relative activities refer to the highest level of extractable radioactivity which was measured for VvGT14a with the racemic citronellol (100 %). Relative specific activity (%) of VvGT15a-c proteins from *Vitis vinifera* towards racemic citronellol and enantiomerically pure *R-* and *S*-citronellol (B). The relative activities refer to the highest level of extractable radioactivity (100 %) which was measured for each protein with racemic citronellol (VvGT15c) or *S*-citronellol (VvGT15a and VvGT15b).

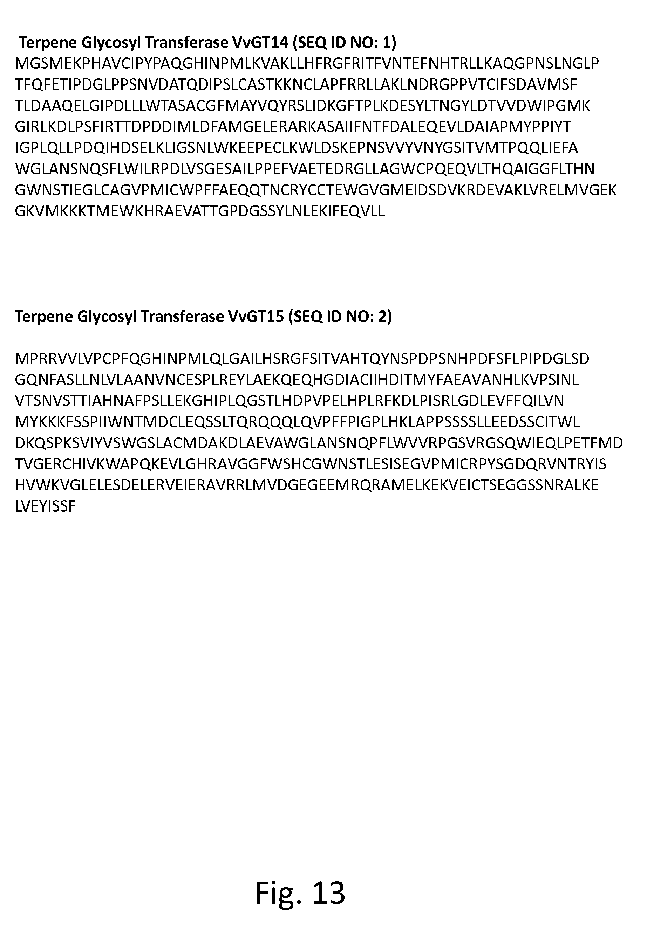

Figure 13 shows the amino acid sequences of terpene glycosyl transferases VvGT14 and VvGT15. (A) Amino acid sequence of terpene glycosyl transferases VvGT14 (SEQ ID NO: 1). (B) Amino acid sequence of terpene glycosyl transferases VvGT15 (SEQ ID NO: 2).

In the following, reference is made to the examples, which are given to illustrate, not to limit the present invention.

### Examples

### Example 1

### Plant Material

*Vitis vinifera* grapevines of cultivars Gewurztraminer 11-18 Gm, Gewurztraminer FR 46-107, White Riesling 239-34 Gm, White Riesling 24-196 Gm and Muscat a petit grains blancs FR 90 were grown. Grape berries, leaves, inflorescences and roots were collected. Sampling was conducted for a total of six dates between 6 and 17 weeks after bloom including berries from pea-size to harvest ripeness. Muscat a petit grains blancs FR 90 was additionally sampled every two weeks from week 4 to week 18 after bloom.

After veraison (the onset of ripening) 100 berries were collected for the determination of ripening related parameters like sugar content. For terpenoid analysis 250 g of berries were stored at -20 °C, while three replicates consisting of ten berries were peeled and skins immediately frozen in liquid nitrogen for subsequent RNA extraction. Roots were obtained from scions of White Riesling 239-34 Gm and Gewurztraminer 11-18 Gm grown in the greenhouse. Leaves were sampled from the same cultivars at the approximate age of one, three and five weeks. In addition, inflorescences four and two weeks before flowering and at full bloom were collected. Samples were stored at -20°C until work-up.

### Chemicals

UDP-[¹⁴C]glucose (300 mCi/mmol, 0.1 mCi/mL) was obtained from American Radio labelled Compounds (St Louis, MO, USA). (*R,S*)-3,7-dimethyl-1,6-octadien-3-ol (linalool) and (*E*)-3,7-dimethyl-2,6-octadien-1-ol (geraniol) were obtained from Roth (Karlsruhe, Germany). (*R*,*S*)-3,7-dimethyl-6-octen-1-ol (citronellol) and pure (*R*)-(+)-13-citronellol were purchased from Sigma Aldrich (Steinheim, Germany). (*Z*)-3,7-dimethyl-2,6-octadien-1-ol (nerol) was purchased from Alfa Aesar (Karlsruhe, Germany). (*R,S*)-3,7-dimethyl-6-octenyl ß-D-glucopyranoside (citronellyl ß-D-glucoside), (*Z*)-3,7-dimethyl-2,6-octadienyl ß-D-glucopyranoside (neryl ß-D-glucoside) and (*E*)-3,7-dimethyl-2,6-octadienyl ß-D-glucopyranoside (geranyl ß-D-glucoside) were synthesized according to the Koenigs-Knorr-procedure, (*R*,*S*)-3,7-dimethyl-1,6-octadienyl ß-D-glucopyranoside (linaloyl ß-D-glucoside), according to a modified Koenigs-Knorr-procedure.

### Sample preparation for metabolite analysis

Grapes berries were peeled and 10 g (fresh weight) of the grape skins were taken for one analysis. In case of root, leaf and inflorescence, 4 g plant material was taken per analysis. The material was frozen in liquid nitrogen, ground and extracted with a mixture of phosphate buffer (0.1 M, pH 7) and 13% ethanol for 24 h under nitrogen with exclusion of light (Jesus Ibarz et al., 2006). 2-Octanol was used as internal standard for the determination of free monoterpenes. For the determination of monoterpenyl ß-D-glucosides, stable isotope dilution analysis (SIDA) was applied, using [²H₂]-citronellyl ß-D-glucoside as a labeled, internal standard. The concentration of the internal standards was adapted for the variety, the tissue and the ripening stage of the plant material. 2-Octanol was added in a range of 0.3 to 6.8 mg/kg plant material, [²H₂]-citronellyl-ß-D-glucoside in a range of 0.1 to 3.5 mg/kg plant material.

To purify the sample, Carrez reagents (Merck Millipore, Darmstadt, Germany) were added (1 mL each) and the sample was then centrifuged at 14500 rpm for 20 min at 5 °C. The supernatant was taken for subsequent solid phase extraction (SPE) to isolate and separate free monoterpenes from glycosidically bound monoterpenes. Therefore, a 200 mg Lichrolut EN column (Merck, Darmstadt, Germany) was conditioned as described (Piñeiro et al., 2004). Free monoterpenols were eluted with dichloromethane and glycosidically bound monoterpenols with methanol.

For GC-MS detection, the dichloromethane fractions were dried with Na₂SO₄, concentrated using nitrogen to 200 µL and analyzed. For LC-MS detection, the methanolic fractions were concentrated under reduced pressure and the residues were dissolved in water/acetonitrile (7/3; v:v). The samples were analyzed by LC-MS.

### Nucleic acid extraction

For total RNA extraction plant material was ground to a fine powder in liquid nitrogen using mortar and pestle. One g of the powder was used for RNA extraction with the CTAB (cetyltrimethylammonium bromide) method following an established protocol (Zeng and Yang, 2002, adapted by Reid et al. 2006) to meet the requirements of different grape tissues. Remaining genomic DNA was digested by DNase I and cleaned up with the High Pure RNA Isolation kit (Roche, Mannheim, Germany).

### Transcription analysis

Transcription analysis was performed using the Genome Lab GeXP Genetic Analysis System (Beckman Coulter, Krefeld, Germany), a multiplex quantitative gene expression analysis system. The gene expression patterns of the *VvGT* genes *VvGT14, VvGT15* and *VvGT16* and five reference genes *(VvActin, hvAP47, hvPP2A, VvSAND,* and *hvTIP41)* were analyzed simultaneously from one sample of total RNA.

Reverse transcription was carried out with the GenomeLab^{™} GeXP Start kit (Beckman Coulter) following the manufacturer's instructions. As an internal control gene KAN^{r} RNA was co-reverse transcribed and subsequently amplified together with the reference genes and genes of interest. The gene specific primers for reverse transcription are chimeric, providing a 19 nt universal tag for the binding of universal reverse primers in the subsequent PCR reaction. The final concentration of the primers ranged from 0.1 nM to 100 nM to compensate for the different transcription levels of the analyzed genes (Table 3). Multiplex PCR reactions were conducted with Thermo-Start DNA Polymerase (Thermo Fisher Scientific, Dreieich, Germany). Each reaction contained 9.3 µL of reverse transcription products as template and 10.7 µL of a PCR reaction mix including gene specific forward primers providing an 18 nt universal tag (Table 3). The universal forward primer is labeled with a fluorescent dye for detection during subsequent capillary electrophoresis. Primer pairs were designed to yield PCR products ranging from 119 bp to 374 bp and differing in size by at least 8 bp. Of each PCR product, 4 µL were separated by capillary electrophoresis using the GenomeLab Genetic Analysis System (Beckman Coulter).

Individual standard curves for each gene in the multiplex were performed with serial two-fold dilutions ranging from 3.91 ng to 500 ng of an RNA mixture from all samples. Raw data were analyzed using the fragment analysis tool. The fragment data of the standard curves and samples were then normalized to the peak area of KAN^{r} RNA with the express analysis tool. Subsequently, the relative signal level of each sample replicate was interpolated from the standard curve. The data was further normalized to the geometric mean of the five reference genes with quant tool. All software for GeXP data analysis was purchased from Beckman Coulter.

### Comparative Sequencing

The reference genome of *Vitis vinifera* sequenced genome PN40024 was used to design gene specific primers in the untranslated regions of the three putative VvGT genes, VvGT14, VvGT15, and VvGT16 using the tool Primer-BLAST. Primers (Table 4) were purchased from Eurofins MWG Operon (Ebersberg, Germany). The cDNA-synthesis was performed with the SuperScript® III First-Strand Synthesis SuperMix (Life Technologies, Darmstadt, Germany) following the manufacturer's instructions. The template for cDNA-synthesis was total RNA, extracted from samples with high transcript-levels of the concerned VvGT gene, as determined by gene expression analysis. The cDNA was used as template in the following PCR-reaction. PCR was performed with Phusion DNA Polymerase (Thermo Fisher Scientific, Dreieich, Germany) using high-fidelity (HF)-buffer and the following thermal cycling conditions: 98 °C for 30 s followed by 32 cycles consisting of 98 °C for 5 s, 60 °C for 5 s and 72 °C for 30 s and a final elongation step of 72 °C for 1 min.

PCR products were gel purified with the Wizard SV Gel and PCR Clean-Up System (Promega, Madison, USA). A-tailing of purified PCR-products was performed with *Taq* DNA Polymerase (Thermo Fisher Scientific). A-tailed PCR-products were ligated into pGEM-T Easy vector (Promega, Madison, USA) and cloned in One Shot TOP10 Chemically Competent *E.Coli* (Life Technologies, Darmstadt, Germany). Plasmids were isolated with the PureYield Plasmid Miniprep System (Promega, Madison, USA) and sequenced with the vector specific primers M13 uni (-21) and M13 rev (-29) on an ABI 3730 capillary sequencer (StarSEQ, Mainz, Germany). Raw data was edited with the FinchTV software (Geospiza, Seattle, USA). Sequences were assembled with SeqMan and aligned with MegAlign (DNASTAR, Madison, USA).

### Construction of expression plasmids

The full-length ORFs of the *VvGT* sequences were subcloned into the pGEM-Teasy vector (Promega, Madison, WI, USA). All genes were amplified with primer introducing BamHI und NotI restriction sites. Subsequently, the genes were cloned *in frame* with the N-terminal GST-tag into the pGEX-4T-1 expression vector (Amersham Bioscience, Freiburg, Germany). The gene identity was confirmed by sequencing (Eurofins MWG Operon, Ebersberg, Germany).

### Heterologous protein expression

Recombinant protein was expressed in *E. coli* BL21 (DE3) pLysS (Novagen, Schwalbach, Germany). Pre-cultures were grown overnight at 37 °C in Luria Bertani medium containing 100 µg/mL ampicillin and 23 µg/mL chloramphenicol. The next day, 1 L Luria Bertani solution containing the appropriate antibiotics was inoculated with 50 mL of the pre-culture. The culture was grown at 37 °C at 160 rpm until OD₆₀₀ reached 0.6-0.8. After cooling the culture to 16 °C, 1 mM IPTG was added to induce protein expression. Cultures were incubated over night at 16-18 °C at 160 rpm. Cells were harvested by centrifugation and stored at -80 °C. Negative controls were carried out with *E. coli* BL21 (DE3) pLysS cells containing the empty expression vector pGEX-4T-1.

### Cell lysis and purification

Recombinant GST-fusion proteins were purified by GST Bind resin (Novagen) following the manufacturer's instructions. Briefly, the cells were re-suspended in the binding buffer containing 10 mM 2-mercaptoethanol. Cells were disrupted by sonication. The crude protein extract was incubated for 2 h with the GST Bind resin to bind GST fusion protein. The recombinant protein was eluted with GST elution buffer containing reduced glutathione and quantified by Bradford solution (Sigma-Aldrich, Steinheim, Germany). The presence of the expressed proteins was confirmed by SDS-PAGE and Western Blot using anti-GST antibody.

### Activity assay and kinetics

In the initial screening, each reaction mixture (200 µL in total) contained Tris-HCl buffer (100 mM, pH 8, 10 mM 2-mercaptoethanol), 37 pmol UDP-[¹⁴C]glucose (0.01 µCi, Biotrend, Köln, Germany), substrate (50 µL of a 1 mg/mL stock solution) and purified protein (0.5-0.8 µg/µL). The reaction mixture was incubated at 30 °C for 18.5 hours. The assays were stopped by adding 1 µL 24% trichloroacetic acid and extracted with 500 µL water-saturated 1-butanol. The organic phase was mixed with 2 mL Pro Flow P+ cocktail (Meridian Biotechnologies Ltd., Epsom, UK) and radioactivity was determined by liquid scintillation counting (LSC, Tri-Carb 2800TR, Perkin Elmer, Waltham MA, USA). Additionally, negative controls without substrate were performed.

After determining the optimal conditions for the best substrate of each gene, the substrate screening was repeated under these conditions.

The kinetic data were determined with increasing concentrations of the substrates (VvGT14a: citronellol, geraniol, 8-hydroxylinalool, linalool, nerol and terpineol; VvGT15a-c: *S-*citronellol, geraniol, 8-hydroxylinalool and nerol; VvGT16: citronellol, geraniol and nerol) from 1 µM to 100 µM (VvGT14a and VvGT15a-c) or 50 µM to 500µM (VvGT16) and a fixed UDP-glucose concentration of 108 µM (100 uM unlabeled UDP-glucose and 8 µM UDP-[¹⁴C] glucose; VvGT14a), 833 µM (825 µM unlabeled UDP-glucose and 8 µM UDP-[¹⁴C] glucose;VvGT15a-c) or 512,5 µM (500 µM unlabeled UDP-glucose and 12,5 µM UDP-[¹⁴C] glucose; VvGT16). The total volume was 40 µL and 0.2 µg (VvGT14a), 0.5 µg (VvGT15a-c) or 5 µg (VvGT16) of purified protein. The measurements were performed under the following conditions. The assays were carried out at 30 °C for 1.5 h using a Tris-HCl buffer (100 mM, 10 mM 2-mercaptoethanol, pH 8.5 for VvGT14a and VvGT16). The assay of VvGT15a-c was performed at 30 °C using a Tris-HCl buffer (100 mM, 10 mM 2-mercaptoethanol, pH 7.5) and 10 min (VvGT15c) or 30 min (VvGT15a-b) of incubation for the best substrates. The amount of the purified enzyme and the incubation time were adapted depending on the counting sensibility. The reaction was stopped by adding 1 µL 24% trichloroacetic acid and glucosides were extracted with 100 µL ethyl acetate. Radioactivity was determined by LSC.

To determine the kinetic data of UDP-glucose, the value of geraniol was fixed (1.25 mM for VvGT15a-c and VvGT16; 0.1 mM for VvGT14a) and UDP-[¹⁴C] glucose was mixed with non-radio labeled UDP-glucose to obtain concentrations ranging from 5 µM to 100 µM (VvGT14a and VvGT15a-c) or 25 µM to 500 µM (VvGT16). The K_{M}- and vₘₐₓ-values were calculated from Lineweaver-Burk plots, Hanes-Woolf plots and non-linear fitting of the experimental data.

### Preparation of aglycone libraries

For the preparation of aglycone extracts 50 g (fresh weight) grape skins or 4 g (fresh weight) inflorescences of Gewurztraminer Gm 11-18 were ground to a fine powder in liquid nitrogen. The isolation of glycosides was carried out as described above (see metabolite analysis). The methanolic fraction, which was obtained after SPE, was enzymatically hydrolysed. Therefore, the dried sample was dissolved in citric-acid buffer (0.1 M, pH 4), 50 mg AR 2000 (an enzyme preparation with glucosidase activity prepared from *Aspergillus niger;* DSM Food Specialties Beverage Ingredients, Delft, Netherlands) was added and incubated for 24 h with exclusion of light. The liberated aglycones were extracted by 20 mL methyl-*tert*-butyl ether and the organic phase was reduced to 1000µL using a gentle stream of nitrogen.

### Activity based profiling using a physiologic aglycone library

Aliquots of this aglycone extract were incubated with UDP-glucose and various VvGT-enzymes. Optimum conditions at 30 °C for 24 hours were applied. Each solution contained 100 µL purified enzyme, 100-150 µL Tris-HCl buffer (100 mM, pH7.5 or 8.5, 10 mM 2-mercaptoethanol), 37 pmol UDP-[¹⁴C]glucose (0.01 µCi) and 50-100 µL extract (dissolved in methyl-*tert*-butylether). The buffer was mixed with the extract and the organic solvent was gently vaporized with nitrogen. The missing volume was adjusted with buffer before the enzyme was added. The reaction was stopped by adding 1 µL 24% trichloroacetic acid and extracted with 500 µL ethyl acetate. After termination of the reaction free aglycones, which were not converted by VvGT14 and VvGT15, were measured via SPME-(Solid Phase Microextraction)-GC-MS. These residual, free aglycones were completely removed by extraction with dichloromethane. Enzymatically formed glucosides were extracted by ethyl acetate, which was removed under nitrogen. The residue was dissolved in methanol and analyzed by LC-MS to detect the generated monoterpenol glucosides. Enzymatic hydrolysis of the glucosides was performed using AR 2000 and 2 mL citric-acid-buffer. After hydrolysis, a 100 µL aliquot was used to detect volatile aglycones via SPME-GC-MS. The remaining solution was extracted with methyl-*tert*-butyl ether and reduced under nitrogen to approximately 200 µL. One µL were measured by GC-MS via liquid injection.

### Enantioselectivity of VvGT14a, VvGT15a-c

To determine the enantioselectivity of VvGT14 and VvGT15 the enantiomeric ratio of glucosidically bound citronellol and linalool was determined by enantioselective GC-MS. Following the incubation, residual citronellol and linalool were completely removed by extraction with dichloromethane. Citronellyl ß-D-glucoside which remained in the aqueous phase was hydrolyzed by HCl (2 mL, 0.1 M, pH 1) for one hour at 100 °C to release citronellol (Skouroumounis and Sefton, 2000). In case of linalyl ß-D-glucoside an enzymatic hydrolysis (AR 2000, citric acid buffer pH 4, 24 h) was applied due to the instability of linalool in acid solutions (Williams et al., 1982). Hydrolysis of a synthetic 1:1 mixture of *R-* and *S*-linalyl ß-D-glucoside revealed that AR 2000 does not discriminate between the two diastereomeric glucosides. After hydrolysis, citronellol and linalool were analyzed by SPME-GC-MS as described above.

### HPLC-MS analysis

For LC-MS analysis of monoterpenyl ß-D-glucosides a Shimadzu LC20AD HPLC system coupled to an API 2000 (Applied Biosystems, AB Sciex, Framingham, USA) triple-quadrupol-MS was used. Data acquisition was performed using Analyst software version 1.6.1. (Applied Biosystems, AB Sciex, Framingham, USA). The column (Phenomenex Gemini-NX 5u C18, 250 x 3 mm, Aschaffenburg, Germany) was eluted with a linear gradient starting at water/acetonitrile (7/3; v:v) containing 0.2% ammonia till 12 min to water/acetonitrile (4/6; v:v; 0.2% ammonia) at 18 min. The column temperature was maintained at 40 °C. The mass spectrometer was operated in ESI-MRM negative ion mode. Nitrogen was used as curtain (setting 20), nebulizing and collision gas (collision energy was - 20 eV). Monoterpenyl ß-D-glucosides were identified by the following, characteristic MRM transitions {LinGlc: *m*/*z* 315→161(Glu), 315→113(Glu); NerGlc: *m*/*z* 315→119(Glu), 315→113(Glu); GerGlc: *m*/*z* 315→119(Glu), 315→113(Glu); CitrGlc: *m*/*z* 317→101(Glu), 317→161(Glu)} (Cole et al., 1989; Domon and Costello, 1988; Salles et al., 1991).

### GC-MS analysis

GC-MS analysis was performed with a Varian GC-450 coupled to a Varian MS-240 ion-trap employing a Phenomenex Zebron ZB-WAXplus column (30 m x 0.25 mm x 0.25 µm, Aschaffenburg, Germany). Helium flow rate was 1 mL/min. The analysis was carried out in split mode (liquid injections) or splitless mode (SPME measurements) with 220 °C injector temperature. EI (electron impact ionization)-MS spectra were recorded from *m*/*z* 40 to 300 (ionization energy 70 eV; trap temperature 170 °C). The oven temperature program was 60 °C (3 min), 10 °C/min up to 250 °C (5 min). In case of SPME measurements, liberated aglycones were isolated for 10 min at 60°C using a fiber coated with a 85 µm film of polyacrylate (SUPELCO, Bellefonte, USA). After extraction the SPME fiber was desorbed for 10 min at 250°C in the injection port of the GC-MS system and the column oven program was carried out as described above. Enantioselective GC-MS analysis was performed with a Varian GC-450 coupled to a Varian MS-240 ion-trap. The column was a DiAcß (heptakis-(2,3-di-O-acetyl-6-O-tert.-butyldimethylsilyl)-ß-cyclodextrin), 26 m x 0.32 mm i.d. with a 0.1 µm film. Helium was used as carrier gas at a flow rate of 1 mL/min, injector temperature was 250 °C with a split ratio of 1/100 (liquid injections), or splitless (SPME measurement). The oven temperature program was 70 °C (3 min), 0.5 °C/min to 130 °C 20 °C/min up to 200 °C (3 min). GC-MS measurements were recorded in full scan mode. Selected ion monitoring (SIM) mode was used for quantification.

### Radio-TLC analysis

Assays containing UDP-[¹⁴C]glucose and aglycone libraries or substrates were performed as described above and subsequently extracted with 500 µL ethyl acetate. The organic solvent was vaporized and the pellet was re-suspended in 10 µL methanol and was applied on Silica Gel 60 F254 plates (Merck, Darmstadt, Germany). The dried plates were developed in a solvent system chloroform:acetic acid:water (50/45/5, v:v:v). Plates were dried and analyzed by digital autoradiograph (digital autoradiograph, EG&G Berthold, Wildbad, Germany).

### Example 2

### Expression analysis - temporal and spatial

All methods mentioned in this example were carried out as described in Example 1.

To identify UGT genes that are likely to contribute to the glucosylation of the terpenols geraniol, nerol, citronellol, α-terpineol, and linalool during grape ripening the *Vitis vinifera* cv. Pinot noir genome was screened for sequences with substantial identity to monoterpene UGTs found in *Arabidopsis thaliana.* Thereby, the putative *hvGT* genes *VvGT14* (VIT_18s0001g06060), *hvGT15* (VIT_06s0004g05780) and *hvGT16* (VIT_03s0017g01130) were selected.

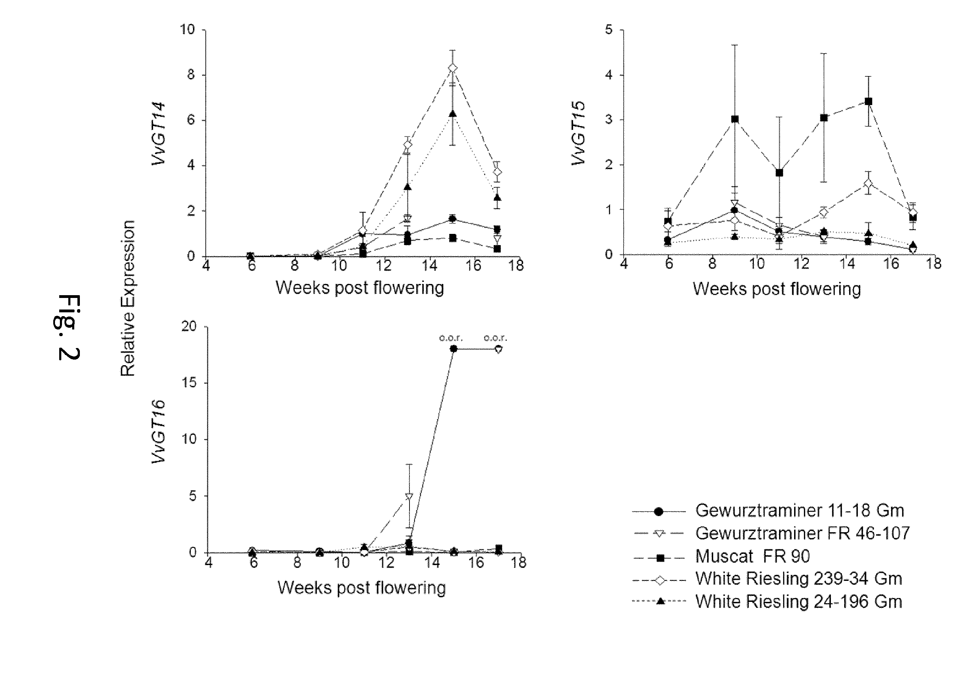

The transcript levels of *VvGT14, VvGT15* and *VvGT16* were analyzed using GeXP profiling in up to five cultivars in three different tissues (Figure 1) and in grape berry exocarp at different developmental stages (Figure 2). In non-berry tissue (inflorescence, leaf and root) *VvGT14* and *15* showed the lowest relative transcript levels of the putative UGT genes but displayed a ripening related expression pattern in berry skins similar to *VvGT16.* Significant amount of *VvGT14-16* mRNA was found in berry exocarp at late stages of berry ripening (Figure 1). Notably, their transcript levels differed considerably between varieties. *VvGT14* was expressed primarily in the two surveyed clones of White Riesling, *VvGT15* in Muscat and *VvGT16* in the two clones of Gewurztraminer. In contrast, *VvGT17-20* transcripts accumulated to comparatively high levels in inflorescence, leaf tissue and immature green berries, but their amount decreased after veraison (the onset of ripening). Expression profiling was also performed for berry skins of Muscat FR 90 in two subsequent years. In 2011, in comparison to 2012, similar relative transcript levels of *VvGT14-15* were reached, but slightly later (2-3 weeks; Figure 8).

The same is true for the ripening related parameters such as sugar content and pH value. Thus, *VvGT14-16* appear to play an important role in grape berry ripening as their expression levels peak after veraison and they are barely expressed in other tissues, except *VvGT16.*

### Example 3

### Metabolite profiling

All methods mentioned in this example were carried out as described in Example 1.

To correlate the expression profiles of putative *UGTs* with terpenyl glucoside concentration metabolite analysis was performed in five cultivars during grape ripening (Table 1). Solid phase extraction was used to isolate free (non-glycosylated) and glycosylated monoterpenes from grape skins (exocarp) of various grapevine cultivars (Gunata et al., 1988; Mateo and Jiménez, 2000). Since grape skins (exocarp) accumulate the majority of terpene metabolites detected in grape berries they were separated from the flesh and extracted (Wilson et al., 1986). The main monoterpenes of grapes (geraniol, nerol, linalool and citronellol) were quantified by GC-MS analysis whereas their non-volatile monoterpenyl glucosides were determined by a stable isotope dilution analysis (SIDA) method using LC-MS. Isotopically labelled internal standards were chemically synthesized.

Grape berries of the *V. vinifera* cultivars differed not only in their amounts of total terpenes, but also in their terpene profiles at different developmental stages (Table 1). Monoterpenols (free and glucosidically bound) were hardly detected (less than 0.25 mg/kg grape skins) in grape exocarp of Gewurztraminer FR 46-107, probably due to impaired monoterpene biosynthesis of this clone. Gewurztraminer 11-18 Gm and Muscat a petits grains blanc FR 90 skins accumulated significant levels of geraniol, citronellol and nerol derivatives (up to 5.5 mg/kg grape skins) and displayed a heterogenous spectrum of monoterpenes at every stage of ripening. Both Riesling clones produced smaller amounts of the metabolites that were mainly observed at weeks 15-17. In general, the highest concentration of free and bound terpenols was found in the late stages of ripening in all investigated cultivars, whereupon geraniol and its ß-D-glucoside were the predominant terpene metabolites. The ratios of the amount of free to glucosidically bound forms of individual monoterpenes varied considerably at week 15 and 17 post flowering. These values provide a first indication of variable UGT activity in different varieties and/or differential preference of the UGTs for their monoterpene substrates.

Notably, the evolution of monoterpenyl ß-D-glucosides in grape exocarp of the Riesling clones (Table 1) correlated well with the expression pattern of *VvGT14* in the same tissue (Figure 2). While significant transcript levels were only detected at week 11 post flowering remarkable levels of the glucosides were not found until week 13. In contrast, the time course of *VvGT15* mRNA levels in Muscat FR 90 coincided with the terpenyl glucoside concentrations in the same clone as considerable amounts of transcripts and glucosides were found throughout weeks 6 to 17 post flowering. At the very late stages of ripening (weeks 15 to 17) expression of *hvGT16* increased strongly in Gewurztraminer 11-18 Gm, a variety that produced a high concentration of geranyl ß-D-glucosides.

Our results revealed a large varietal variation in the content of terpenes and their glycosidic derivatives (Table 1). Gewurztraminer 11-18 Gm and Muscat FR 90, two genotypes that are rich in monoterpenes, also accumulated high levels of the glucosides that can also be detected at early stages of berry set (six weeks post flowering). Besides, the ratio of free to glycosylated terpenes differed for the varieties and sampling dates indicating differential glycosylation activities during berry ripening in the diverse genotypes. As terpenoids and their glucosides were accumulating in berry skins after veraison, the strong expression of *VvGT14, VvGT15* and *VvGT16* during late stages of berry ripening suggested an important role during this period and led to in-depth analysis of these genes (Figure 2). Notably, *VvGT14* transcript levels detected in both White Riesling clones correlated well with the late formation of monoterpenyl glucosides in the same varieties (Table 1 and Figure 2) whereas expression level of *VvGT15* in Muscat FR 90 showed a similar time course pattern as the amounts of the glucosides. In Muscat FR 90 transcription of *VvGT15* and production of terpenyl glucosides started already at 6 to 9 weeks post flowering and remained at a high level. Besides, in Gewurztraminer 11-18 Gm, *VvGT15* expression peaked at 9 weeks post flowering and then decreased continuously although monoterpenol derivatives accumulated to high levels in grape skin. However, compensation of VvGT15 activity by VvGT16 was assumed as *hvGT16* expression levels even exceeded the range of GeXP quantification at the late stages of ripening.

### Example 4

### Heterologous expression of VvGT14, VvGT15 and VvGT16 and enzymatic activity

All methods mentioned in this example were carried out as described in Example 1.

The alleles of *VvGT14a-c, 15a-c and 16* were isolated from *V. vinifera* cultivars and cloned in the expression vector pGEX-4T-1. The recombinant proteins were expressed with an N-terminal GST-tag, affinity purified and verified by SDS-PAGE and Western Blot using GST-specific antibody. Enzyme activity studies were performed with UDP-[¹⁴C]glucose and various putative substrates (terpenols, flavonoids and different mono-alcohols) that are known to be glycosidically bound and present in grapes (Gunata et al., 1988).

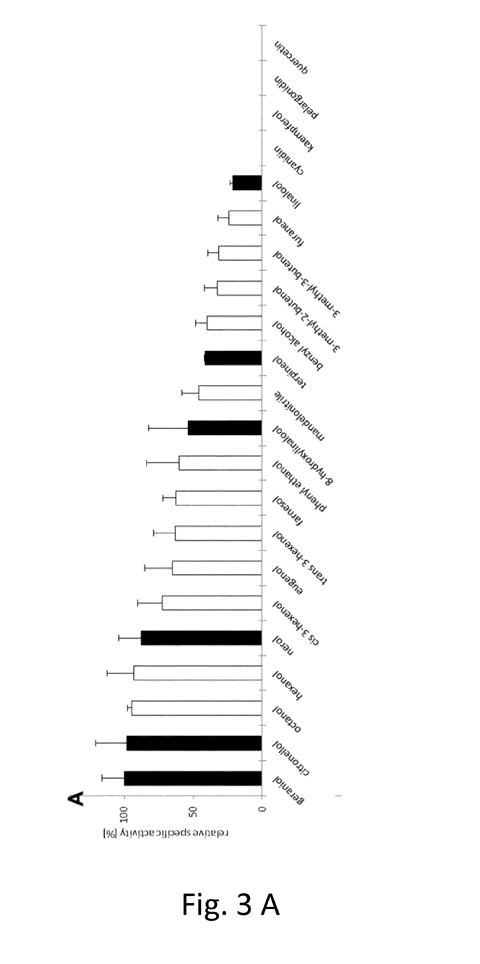

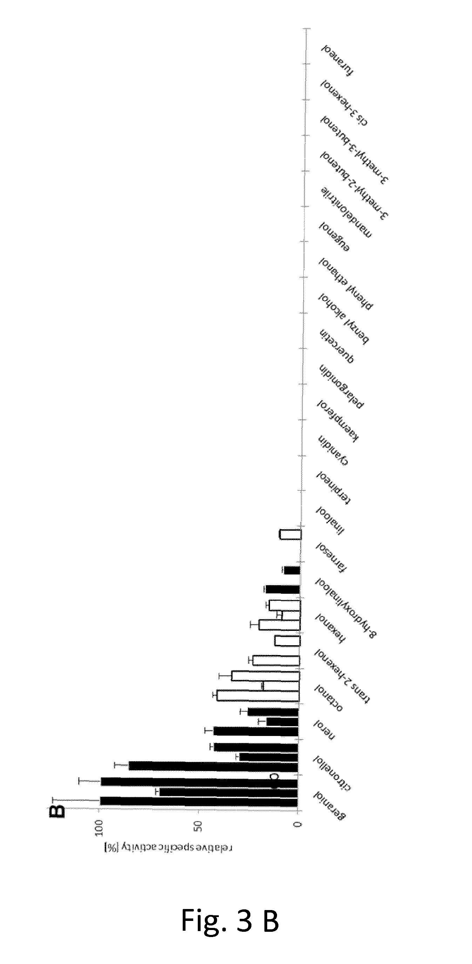

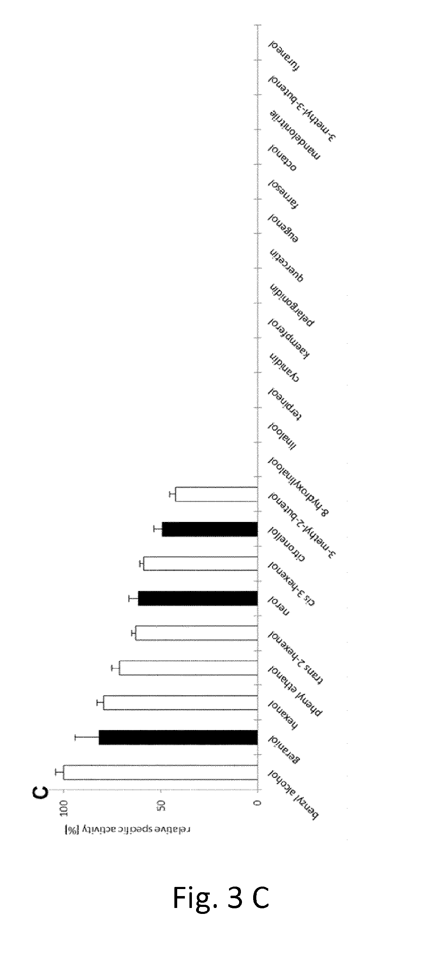

Recombinant VvGT14a, VvGT15a-c and VvGT16 converted several of the tested substrates (Figure 3). VvGT14a preferred geraniol and citronellol but also efficiently (> 80% relative activity) glucosylated nerol, hexanol and octanol. Additionally, VvGT14a showed catalytic activity towards further monoterpenes (terpineol, 8-hydroxylinalool, linalool), short-chain mono-alcohols (3-methyl-2-butenol, 3-methyl-3-butenol, *cis* 3- and *trans* 2-hexenol), benzyl alcohol, phenylethanol, eugenol, farnesol, mandelonitrile, and furaneol. The tested anthocyanidins and flavonoids (cyanidin, pelargonodin, quercetin, and kaempferol,) were not converted at all (< 1%). The three active proteins VvGT15a-c showed a more limited substrate spectrum and glucosylated primarily geraniol, citronellol, nerol, octanol, and hexanol. VvGT15a and c were also able to use 8-hydroxylinalool and *trans* 2-hexenol as acceptor molecule and VvGT15a had low activity for farnesol. Other tested substrates were not converted. VvGT16 showed highest activity towards benzyl alcohol, geraniol and hexanol (> 80% relative activity). Additionally, the protein transformed the terpenoids citronellol and nerol as well as phenylethanol, 3-methyl-2-butenol, *trans* 2-hexenol and *cis* 3-hexenol.

The K_{M} and k_{cat} values of VvGT14a for geraniol (9 µM; 0.02 sec⁻¹), citronellol (9 µM; 0.02 sec⁻¹), nerol (10 µM; 0.02 sec⁻¹) and UDP-glucose (16 µM; 0.03 sec⁻¹) were alike (Table 2) and resembled the kinetic data known in the literature for VvGT1 and CaUGT2 *(Catharanthus roseus)* for their natural substrates quercetin and curcumin, respectively. The data were also similar to those of VvGT5, VvGT6 and UGT71G1 from *Medicago truncatula* for the conversion of quercetin as known from the literature. Hence, the specificity constants (k_{cat}/K_{M}) identified the monoterpenol as most probable *in vivo* substrates of VvGT14a. However, the Michaelis constant for terpineol, 8-hydroxylinalool and linalool were 3 to 5-fold higher while the turnover numbers were 10 to 100-fold lower than for geraniol, nerol and citronellol. Thus, the enzyme efficiency values made it unlikely that they are converted by VvGT14a, *in planta.*

The sequence comparison of the active VvGT14a allele with the two inactive alleles showed one point mutation at position 391 (P391L) in VvGT14b and a deletion of 21 aa at position 165 in VvGT14c rendering them inactive (Figure 11). Proline in position 391 is located in the PSPG motif plant secondary product glycosyltransferase) and is quite conserved among UGTs. The exchange of proline to leucine in VvGT14b leads to an inactive enzyme and has not been reported, yet.

The allelic forms of VvGT15a-c showed a similar substrate spectrum (Figure 3). Remarkably, these alleles had a distinct preference for the monoterpenes geraniol, citronellol and nerol as comparison with the accepted substrates of VvGT14a clearly demonstrated. The kinetics identified geraniol as superior substrate (Table 2). The turnover numbers of VvGT15a-c for the glucosylation activity of geraniol (0.12, 0.1, and 0.17 sec⁻¹, respectively) were 3 to 6-fold higher and the Michaelis constants (63, 81, and 43 µM, respectively) about 2-fold greater than the k_{cat} and K_{M} values for nerol and *S*-citronellol. This resulted in a highest enzyme specificity constant of VvGT15a-c for geraniol and confirmed the data of the substrate screening (Figure 3) whereas allele c was the most effective (k_{cat}/ K_{M} 3.9 sec⁻¹ mM⁻¹). All three alleles showed a 30 to 40- fold lower turnover number for 8-hydroxylinalool compared to geraniol, although the K_{M} value was similar to the ones obtained for *S*-citronellol and nerol.

VvGT16 glucosylated monoterpenols, some short-chained and aromatic alcohols, albeit with low efficiency (Figure 3, Table 2). Interestingly, benzyl alcohol, an alcohol which has been frequently detected in hydrolysates of glycosides from grape, showed the highest relative activity. However, the low k_{cat}/K_{M} values argue against a role of VvGT16 in the glucosylation of these compounds *in planta.*

The formation of monoterpenyl glucosides was confirmed by LC-MS analysis in comparison with chemically synthesized glucosides (Figure 4). The retention times and fragmentation patterns of the reference material and products formed by VvGT14a, 15a and 16 were identical and in accordance with the proposed fragmentation mechanism (Domon and Costello, 1988; Cole et al., 1989; Salles et al., 1991).

Besides, selected glucosides of the transformed terpenoids were visualized by radio-TLC (Figures 9 and 10). The extracted radioactivity of the enzyme assays consisted exclusively of the terpenyl mono-glucosides, except when 8-hydroxylinalool was used. It seemed that this monoterpene diol is glucosylated at both hydroxyl groups as two spots, presumably the mono- and diglucoside appeared on the radio-TLC plate. The two allelic enzymes VvGT14b and 14c were unable to glycosylate any of the tested substrates. The alignment of the three VvGT14 alleles showed that VvGT14c has an internal deletion of 21 amino acids at position 165 (Figure 11), while VvGT14a and b differed in a single position (P391L).

### Example 5

### Enantioselectivity of VvGT14a, VvGT15a-c and VvGT16

All methods mentioned in this example were carried out as described in Example 1.

Grape berries accumulate free and bound *S*-citronellol and *S*-linalool, albeit in lower levels than nerol and geraniol (Table 1). To elucidate the enantiomeric preference of VvGT14a and VvGT15a, racemic citronellol was used as substrate and racemic linalool was transformed by VvGT14a. Chiral phase GC-MS analysis of liberated citronellol after acid hydrolysis of citronellyl ß-D-glucoside demonstrated no enantio-discrimination by VvGT14a and 15a if the reaction mixture is incubated for a prolonged time (24 h; Figure 7). Nevertheless, VvGT15a-c and VvGT14a preferred S- over *R*-citronellol (1/0.4 and 1/0.8, respectively) when choosing short incubation times for kinetic assays whereas VvGT16 transformed *R*- and *S*-citronellol with the same efficiency in radiochemical assays (Figure 12). Accordingly, the kinetic data for VvGT15a-c were calculated for *S*-citronellol (Table 2).

Furthermore, liberated linalool, after enzymatic hydrolysis of linaloyl ß-D-glucoside (formed by VvGT14a), showed a slight enrichment of the *R*-enantiomer (Figure 7). It is important to mention that the hydrolysis of "racemic" (1:1 diasteromeric mixture) linaloyl ß-D-glucoside by AR 2000 revealed no enantio-discrimination by the action of this enzyme, which confirmed the results of previous works in the literature. Thus, VvGT14a and VvGT15a-c show low enantioselectivity towards the *S*-enantiomer of citronellol and VvGT14a towards *R-*linalool during short-term assays. The enantiomers of racemic monoterpenes react with different reaction rates in the enzymatic reactions with a VvGT14a and VvGT15a-c.

### Example 6

### Biochemical characterization of VvGT14a, VvGT15a-c and VvGT16

All methods mentioned in this example were carried out as described in Example 1.

The assay conditions were optimized for the conversion of geraniol to determine the kinetic constants of the active enzymes. The highest activity of VvGT14a, 15a-c and 16 was found in Tris-HCl buffer (pH 8.5, 7.5 and 8.5, respectively) at 30 °C. The product formation of VvGT14a (0.2 µg purified enzyme) was linear for at least 90 minutes. K_{M} and k_{cat} values were obtained for geraniol, citronellol, nerol, terpineol, 8-hydroxylinalool, and linalool with a constant UDP-glucose level (108 µM) and for UDP-glucose with a fixed geraniol concentration (100 µM; Table 2). The kinetic parameters were determined from a hyperbolic Michaelis-Menten saturation curves. Due to the low conversion rates of 8-hydroxylinalool, terpineol and linalool, the amount of purified enzyme was increased (2, 2, and 10 µg protein, respectively).

Interestingly, the K_{M} and k_{cat} value of VvGT14a for citronellol. geraniol, nerol and UDP-glucose was quite similar (K_{M} 9-10 µM; k_{cat} 0.02 sec⁻¹; k_{cat}/K_{M} 2.0-2.6 sec⁻¹ mM⁻¹), while the kinetic data for 8-hydroxylinalool, terpineol and linalool explained the significantly lower enzyme activity towards these substrates. The kinetic data of VvGT15a-c (0.5 or 1 µg purified enzyme) were maintained for geraniol, *S*-citronellol, nerol and 8-hydroxylinalool with a fixed UDP-glucose amount (833 µM) and for UDP-glucose with a constant geraniol concentration (1.25 mM; Table 2). The formation of geranyl, neryl, and citronellyl ß-D-glucoside was linear for at least 10 minutes (VvGT15c) and 20 minutes (VvGT15a and b), but was extended for 8-hydroxylinaloyl ß-D-glucoside up to 30 min (VvGT15c) and 60 min (VvGT15a and b). The kinetic data confirmed the high enzymatic activity of the VvGT15 alleles towards geraniol and UDP-glucose. The K_{M} and k_{cat} value for *S*-citronellol and nerol was similar, whereas 8-hydroxylinalool was a poor substrate. Furthermore, the data illustrated that VGT15c is superior to VvGT15a and b. Kinetics of VvGT16 were calculated for geraniol, citronellol and nerol (Table 2). Product formation of VvGT16 (5 µg purified protein) was linear for at least 4 hours. K_{M} and k_{cat} values were obtained for the monoterpenes with a constant UDP-glucose level (512.5 µM) and for UDP-glucose with a fixed geraniol concentration (1.25 mM). VvGT16 exhibited the lowest enzymatic activity towards the substrates of the tested UGTs.

### Example 7

### Identification of the natural substrates of VvGT14a, VvGT15a-c and VvGT16

All methods mentioned in this example were carried out as described in Example 1.

To reveal the natural substrates of VvGT14a, VvGT15a-c and VvGT16 (Figure 6), glycosides were isolated by solid phase extraction from grape skins (Gewurztraminer 11-18 Gm) and blooms (Muscat FR90) as they showed highest expression levels of the target genes. An aglycone library of the two tissues was obtained by enzymatic hydrolysis of the glycosides followed by liquid-liquid extraction of the released alcohols and acids. This physiologic library which contained potential natural substrates of UGTs was screened with recombinant VvGTs and either radiochemically labeled or unlabeled UDP-glucose. The formed glycosides were separated by thin layer chromatography (TLC) and visualized by radiodetection whereas identification could be achieved by LC-MS and NMR analysis and, after hydrolysis, by GC-MS and LC-MS (Figure 6).

Initially, the aglycone extracts were incubated with the purified recombinant enzymes (VvGT14a, 15a, and 16) using radiolabeled UDP-[¹⁴C] glucose. Formed products were extracted; radioactivity was quantified by liquid scintillation counting and analyzed by radio-TLC (Figure 7). Screening of the aglycone library obtained from grape skins by VvGT14a and 15a yielded products that showed identical chromatographic properties as geranyl ß-D-glucoside. Enzymatic hydrolysis of the glucosides formed by VvGT14a liberated a substantial amount of geraniol but also remarkable quantities of citronellol, nerol, benzyl alcohol, phenylethanol, and linalool (Figure 7). A similar result was gained with VvGT15a. VvGT16 did not form a visible product neither by using the berry nor from the bloom extracts. The extract from bloom was only tested with VvGT16. Thus, the aglycone library clearly enabled the detection and identification of the natural substrates of VvGT14a and VvGT15a and confirmed the role of these enzymes during grape berry ripening.

VvGT14a and VvGT15a-c readily formed radiolabeled products when incubated with an aglycone library obtained from grape skins of Gewurztraminer 11-18 Gm (Figure 7A). Geranyl and minor amounts of citronellyl and neryl glucoside (as their free alcohols after hydrolysis) were identified as products of VvGT14a and VvGT15a-c (Figure 7B). In contrast, VvGT16 did not form a glucosylated product although expression level of *VvGT16* and amounts of monoterpenyl glucosides were extraordinary high in berry skins in the late stages of ripening and blooms (Gewurztraminer 11-18 Gm). However, additional UDP-sugars, besides UDP-glucose, were not tested as putative donor molecules and the possible formation of di- and triglycosides of one aglycone was not taken into account (Gunata et al., 1988).

Thus, by using alternative UDP-sugars for the screening of the libraries, novel glycosides can be formed and identified which also occur in nature. In addition, combination of different UDP-sugars with a diversity of recombinant UGTs would also yield aglycones attached to di- and triglycosides. Last but not least, the method can be applied in high-throughput screens. However, insufficient release of aglycones by acidic and enzymatic hydrolysis and instability of liberated aglycones are two drawbacks which have to be kept in mind. Moreover, the obtained glycosodic extract, which is used for formation of the aglycone library, contains glycosides from various cellular compartments like cytosol and vacuole. Because it is known that glycosyltranferases are usually located in the cytosol, it cannot be ruled out that in an *in vivo* scenario some aglycones and glycosyltranferases do not come into contact in intact cells. Nevertheless, aglycone libraries as described have great potential to unravel the physiologic substrates of a wide range of UGTs and thus to clarify their biological roles.

While VvGT14a and VvGT15a-c preferentially glucosylated *S*-citronellol in short time assays enantioselectivity of these enzymes for citronellol was not observe in long term studies

(Figure 5; Figure 12). This effect is characteristic for studies on the kinetic resolution of racemates. The reaction slows down at 50% conversion, when the fast reacting enantiomer is almost consumed and only the slow reacting counterpart is gradually transformed. Thus, the enantiomeric excess of the product peaks at 50% transformation and then decreases slowly but steadily. In contrast, VvGT14a preferred *R*- over *S*-linalool even in long term assays. The slight preference for *R*-linal*o*ol explained the previously observed enrichment of this enantiomer in the glycosidically bound fraction of linalool in Morio Muskat and Muscat Ottonel berries relative to the free fraction. Hence, the moderate enantioselectivities of VvGT14a and 15a-c were in accordance with their proposed biological role as the availability of highly enriched *S*-citronellol and *S*-linalool mainly determined the diastereomeric ratio of the glucosidic product.

The features of the present invention disclosed in the specification, the claims, and/or in the accompanying drawings may, both separately and in any combination thereof, be material for realizing the invention in various forms thereof.

| **Table 1**. Amounts of free Monoterpenes and Monoterpene-ß-D-Glucosides in Grape Skins during Grape Ripening | | | | | | | | |
|---|---|---|---|---|---|---|---|---|
| Weeks post flowering | | | 6 | 9 | 11 | 13 | 15 | 17 |
| White Riesling 239-34 Gm | Linalool | free | n.d. | n.d. | n.d. | n.d. | 0.13 ±0.18 | n.d. |
| | | ß-D-Glucosides | n.d. | n.d. | n.d. | 0.10 ±0.08 | 0.17 ±0.01 | 1.71 ±0.54 |
| | Nerol | free | n.d. | n.d. | n.d. | n.d. | n.d. | n.d. |
| | | ß-D-Glucosides | n.d. | n.d. | n.d. | n.d. | n.d. | 0.05 ±0.03 |
| | Geraniol | free | n.d. | n.d. | 0.09 ±0.01 | 0.25 ±0 | 0.26 ±0.15 | n.d. |
| | | ß-D-Glucosides | n.d. | n.d. | n.d. | n.d. | 0.06 ±0.02 | 0.14 ±0.06 |
| | Citronellol | free | n.d. | n.d. | 0.03 ±0.01 | 0.05 ±0.01 | n.d. | n.d. |
| | | ß-D-Glucosides | n.d. | n.d. | n.d. | 0.03 ±0.02 | n.d. | n.d. |
| White Riesling 24-196 Gm | Linalool | free | n.d. | n.d. | n.d. | n.d. | 0.11 ±0 | n.d. |
| | | ß-D-Glucosides | n.d. | n.d. | n.d. | 0.07 ±0.02 | 0.27 ±0.02 | 0.61 ±0.1 |
| | Nerol | free | n.d. | n.d. | n.d. | n.d. | 0.08 ±0 | n.d. |
| | | ß-D-Glucosides | n.d. | n.d. | n.d. | n.d. | 0.01 ±0.01 | n.d. |
| | Geraniol | free | 0.11 ±0.03 | 0.06 ±0 | 0.17 ±0 | 0.24 ±0.02 | 0.48 ±0.01 | 0.67 ±0.04 |
| | | ß-D-Glucosides | n.d. | n.d. | n.d. | 0.02 ±0 | 0.07 ±0.02 | 0.14 ±0.02 |
| | Citronellol | free | 0.05 ±0 | n.d. | n.d. | 0.06 ±0.02 | 0.09 ±0.01 | n.d. |
| | | ß-D-Glucosides | n.d. | n.d. | n.d. | n.d. | n.d. | n.d. |
| Gewurztraminer FR 46-107 | Linalool | free | - | n.d. | n.d. | n.d. | - | n.d. |
| | | ß-D-Glucosides | - | n.d. | n.d. | n.d. | - | n.d. |
| | Nerol | free | - | n.d. | n.d. | n.d. | - | n.d. |
| | | ß-D-Glucosides | - | n.d. | n.d. | n.d. | - | 0.01 ±0.01 |
| | Geraniol | free | - | n.d. | 0.11 ±0.01 | n.d. | - | 0.20 ±0.01 |
| | | ß-D-Glucosides | - | 0.02 ±0.02 | n.d. | 0.01 ±0.002 | - | 0.03 ±0.04 |
| | Citronellol | free | - | n.d. | n.d. | n.d. | - | n.d. |
| | | ß-D-Glucosides | - | n.d. | n.d. | n.d. | - | n.d. |
| Gewurztraminer 11-18 Gm | Linalool | free | n.d. | n.d. | n.d. | n.d. | n.d. | n.d. |
| | | ß-D-Glucosides | n.d. | 0.1 ±0.04 | n.d. | n.d. | n.d. | n.d. |
| | Nerol | free | n.d. | n.d. | 0.13 ±0.02 | 0.71±0.04 | 1.25 ±0.15 | 1.75 ±0.05 |
| | | ß-D-Glucosides | n.d. | n.d. | 0.06 ±0.01 | 0.23± 0.11 | 0.72 ±0.22 | 0.87 ±0.2 |
| | Geraniol | free | 0.44 ±0.06 | 0.88 ±0.06 | 0.81 ±0.07 | 2.62 ±0.38 | 3.47 ±0.66 | 5.26 ±0.52 |
| | | ß-D-Glucosides | 0.08 ±0 | 0.04 ±0.01 | 0.18 ±0.01 | 0.55 ± 0.16 | 1.72 ±0.18 | 1.53 ±0.4 |
| | Citronellol | free | 0.12 ±0.03 | 0.31 ±0.01 | 0.27±0.02 | 0.49±0.01 | 0.48 ±0.1 | 0.51 ±0.02 |
| | | ß-D-Glucosides | 0.09 ±0.01 | 0.08 ±0.02 | 0.12 ±0.01 | 0.21 ±0.06 | 0.48 ±0.02 | 0.29±0.06 |
| Muscat a petits grains blanc FR 90 | Linalool | free | 0.06 ±0.02 | n.d. | n.d. | 0.13 ±0.02 | 3.03 ±0.01 | 0.62 ±0.12 |
| | | ß-D-Glucosides | 0.11 ±0 | 0.12 ±0.03 | n.d. | 0.05 ±0.01 | 0.27 ±0.10 | 0.37 ±0.16 |
| | Nerol | free | n.d. | n.d. | 0.16 ±0.03 | 0.31 ±0.04 | 0.78 ±0.15 | 0.94 ±0.05 |
| | | ß-D-Glucosides | 0.03 ±0 | 0.04 ±0.02 | n.d. | 0.09 ±0.02 | 0.47 ±0.05 | 0.57 ±0.10 |
| | Geraniol | free | 0.18 ±0.04 | 0.53 ±0.06 | 1.64 ±0.62 | 1.00 ±0.21 | 1.20 ±0.21 | 1.21 ±0.13 |
| | | ß-D-Glucosides | 0.03 ±0 | 0.07 ±0.03 | 0.05 ±0.03 | 0.09 ±0.02 | 0.45 ±0.09 | 0.24 ±0.08 |
| | Citronellol | free | 0.09 ±0.03 | 0.11 ±0.01 | 0.20 ±0.06 | 0.15 ±0.01 | 0.21 ±0 | 0.23 ±0 |
| | | ß-D-Glucosides | n.d. | 0.07 ±0.04 | 0.04 ±0.02 | 0.04 ±0.01 | 0.12 ±0.05 | n.d. |
| Plant material was prepared and analyzed as described in Methods. Grapes were collected during grape ripening at the indicated weeks post flowering. n.d.: not detected, -: not determined, Amounts are listed in mg/kg grape skins. n=2 | | | | | | | | |

| **Table 2**. Kinetics of VvGT14,15a-c and 16. | | | | |
|---|---|---|---|---|
| enzyme | Substrate | K_{M} [µM] | k_{cat} [sec⁻¹] | k_{cat}/K_{M} [sec⁻¹ mM⁻¹] |
| VvGT14 | | | | |
| | citronellol (rac) | 9 ± 0.3 | 0.02 | 2.5 |
| | Geraniol | 9 ± 1.2 | 0.02 | 2.6 |
| | Nerol | 10 ± 0.7 | 0.02 | 2.0 |
| | 8-hydroxylinalool | 48 ± 2.0 | 0.002 | 0.03 |
| | Terpineol | *33* ± 4.4 | 0.003 | 0.1 |
| | linalool (rac) | 47 ± 0.1 | 0.0003 | 0.01 |
| | UDP-glucose | 16 ± 0.03 | 0.03 | 1.6 |

| VvGT15a | | | | |
|---|---|---|---|---|
| | *S*-citronellol | 29 ± 3.0 | 0.02 | 0.9 |
| | Geraniol | 63 ± 2.4 | 0.12 | 1.9 |
| | Nerol | 48 ± 1.7 | 0.04 | 0.7 |
| | 8-hydroxylinalool | 32 ± 1.4 | 0.003 | 0.1 |
| | UDP-glucose | 49 ± 12.0 | 0.18 | 3.7 |

| VvGT15b | | | | |
|---|---|---|---|---|
| | *S*-citronellol | 55 ± 1.3 | 0.03 | 0.6 |
| | Geraniol | 81 ± 1.0 | 0.10 | 1.2 |
| | Nerol | 40 ± 3.7 | 0.03 | 0.8 |
| | 8-hydroxylinalool | *33* ± 1.8 | 0.003 | 0.1 |
| | UDP-glucose | 43 ± 1.0 | 0.17 | 4.1 |

| VvGT15c | | | | |
|---|---|---|---|---|
| | *S*-citronellol | 20 ± 1.6 | 0.03 | 1.8 |
| | Geraniol | 43 ± 0.7 | 0.17 | 3.9 |
| | Nerol | 28 ± 0.9 | 0.06 | 2.2 |
| | 8-hydroxylinalool | 17 ± 0.2 | 0.004 | 0.3 |
| | UDP-glucose | 51 ± 1.6 | 0.26 | 5.0 |

| VvGT16 | | | | |
|---|---|---|---|---|
| | citronellol (rac) | 108 ± 2.5 | 0.008 | 0.07 |
| | Geraniol | 355 ± 14 | 0.015 | 0.04 |
| | Nerol | 118 ± 4.7 | 0.009 | 0.08 |
| | UDP-glucose | 149 ± 10 | 0.013 | 0.09 |

**Table 3. Gene specific primers used for GeXP. All primers are chimeric containing a universal tag sequence at their 5'-end (uncapitalized). The final concentration of each reverse primer in the GeXP reverse transcription reaction is given under conc. The CRIBI-Genomics accession numbers (http://genomes.cribi.unipd.it) are given under locus ID.**

| **gene** | **locus ID** | **primer** | **sequence** | **conc.** | **product** | **SEQ ID NO** |
|---|---|---|---|---|---|---|
| VvActin | VIT_04s0044g00580 | for | aggtgacactatagaataCTTGCATCCCTCAGCACCTT | | | SEQ ID NO: 3 |
| VvActin | VIT_04s0044g00580 | rev | gtacgactcactatagggaTCCTGTGGACAATGGATGGA | 0.1 nM | 119 bp | SEQ ID NO: 4 |
| VvAP47 | VIT_02s0012g00910 | for | aggtgacactatagaataTGTTGTTGAGGCTGTTGCGCTGT | | | SEQ ID NO: 5 |
| VvAP47 | VIT_02s0012g00910 | rev | gtacgactcactatagggaAGGCCCTCTCTCCTCCAACCAA | 1 nM | 304 bp | SEQ ID NO: 6 |
| VvPP2A | VIT_01s0011g03280 | for | aggtgacactatagaataAGGGTTGTGCCACACTGGGC | | | SEQ ID NO: 7 |
| VvPP2A | VIT_01s0011g03280 | rev | gtacgactcactatagggaGCAACCATGTAGCGAACACGCC | 2nM | 152 bp | SEQ ID NO: 8 |
| VvSAND | VIT_06s0004g02820 | for | aggtgacactatagaataACCCCTTTGCTCGGAGGAACAGA | | | SEQ ID NO: 9 |
| VvSAND | VIT_06s0004g02820 | rev | gtacgactcactatagggaACCTGAAGCTTGCCTTGTCGC | 2nM | 166 bp | SEQ ID NO: 10 |
| VvTIP41 | VIT_03s0091g00270 | for | aggtgacactatagaataGCTACCGGAAACCAGCGGGC | | | SEQ ID NO: 11 |
| VvTIP41 | VIT_03s0091g00270 | rev | gtacgactcactatagggaGCAATCCATGCCGTCCATCCGT | 1 nM | 144 bp | SEQ ID NO: 12 |
| VvGT14 | VIT_18s0001g06060 | for | aggtgacactatagaataTGTGGCTTCATGGCCTATGTGCA | | | SEQ ID NO: 13 |
| VvGT14 | VIT_18s0001g06060 | rev | gtacgactcactatagggaCCGGAAGTAGCTGAAGAGGACCA | 12.5 nM | 374 bp | SEQ ID NO: 14 |
| VvGT15 | VIT_06s0004g05780 | for | aggtgacactatagaataTCCGGACGGCTTATCTGATGGCC | | | SEQ ID NO: 15 |
| VvGT15 | VIT_06s0004g05780 | rev | gtacgactcactatagggaTGAGGGGATGAAGCTCAGGCACTG | 50 nM | 363 bp | SEQ ID NO: 16 |
| VvGT16 | VIT_03s0017g01130 | for | aggtgacactatagaataGCAGTTTCGCCCTCTTATTGATGGA | | | SEQ ID NO: 17 |
| VvGT16 | VIT_03s0017g01130 | rev | gtacgactcactatagggaAGCTTTGGAAGCACTGTCCGTT | 50 nM | 248 bp | SEQ ID NO: 18 |

**Table 4. Primers used in PCR for subsequent cloning and sequencing. The CRIBI-Genomics accession numbers (http://genomes.cribi.unipd.it) are given under locus ID.**

| **gene** | **locus ID** | **primer** | **sequence** | **SEQ ID NO** |
|---|---|---|---|---|
| VvGT14 | VIT_18s0001g06060 | for | AATGGGTTCCATGGAGAAGCC | SEQ ID NO: 19 |
| VvGT14 | VIT_18s0001g06060 | rev | GGAAAGTTGAGATCTAGAGAAGC | SEQ ID NO: 20 |
| VvGT15 | VIT_06s0004g05780 | for | TCACCTCTCAACTCTTCCACG | SEQ ID NO: 21 |
| VvGT15 | VIT_06s0004g05780 | rev | GAGTTTGGTTGTACACAACTCTG | SEQ ID NO: 22 |
| VvGT16 | VIT_03s0017g01130 | for | TCCTCCCCAGAGTGCAAG | SEQ ID NO: 23 |
| VvGT16 | VIT 03s0017g01130 | rev | TGACCAACTCCTATAACAG | SEQ ID NO: 24 |

### References

Bowles, D., Lim, E.K., Poppenberger, B., Vaistij, F.E. (2006) Glycosyltransferases of lipophilic small molecules. Annu. Rev. Plant Biol. 57: 567-597.
Cole, R.B., Tabet, J.C., Salles, C., and Crouzet, J. (1989). Structural "memory effects" influencing decompositions of glucose alkoxide anions produced from monoterpene glycoside isomers in tandem mass spectrometry. Rapid Commun. Mass Spectrom. 3: 59-61.
Domon, B. and Costello, C.E. (1988). A systematic nomenclature for carbohydrate fragmentations in FAB-MS/MS spectra of glycoconjugates. Glycoconjugate 5: 397-409.
Gunata, Z., Bitteur, S.M., Brillouet, J.-M., Bayonove, C.L., and Cordonnier, R. (1988). Sequential enzymic hydrolysis of potenially aromatic glycosides from grape. Carbohydr. Res. 184: 139-149.
Jesus Ibarz, M., Ferreira, V., Hernández-Orte, P., Loscos, N., and Cacho, J. (2006). Optimization and evaluation of a procedure for the gas chromatographic-mass spectrometric analysis of the aromas generated by fast acid hydrolysis of flavor precursors extracted from grapes. J. Chromatogr. A 1116: 217-229.
Mateo, J., and Jiménez, M. (2000). Monoterpenes in grape juice and wines. J. Chromatogr. A 881: 557-567.
Piñeiro, Z., Palma, M., and Barroso, C.G. (2004). Determination of terpenoids in wines by solid phase extraction and gas chromatography. Anal. Chim. Acta 513: 209-214.
Reid, K.E., Olsson, N., Schlosser, J., Peng, F., and Lund, S.T. (2006). An optimized grapevine RNA isolation procedure and statistical determination of reference genes for real-time RT-PCR during berry development. BMC Plant Biol. 6: 27.
Salles, C., Jallageas, J.-C., Beziat, Y., and Cristau, H.-J. (1991). Synthesis of 4- and 10-deuterated neryl and geranyl-β-D-glucosides and their use in corroboration of a mechanism proposed for the fragmentation of heterosides in tandem mass spectrometry. J. Label. Compd. Radiopharm. 31: 11-22.
Skouroumounis, G.K. and Sefton, M.A. (2000). Acid-catalyzed hydrolysis of alcohols and their β-D-glucopyranosides. J. Agric. Food Chem. 48: 2033-2039.
Williams, P.J., Strauss, C.R., Wilson, B., and Massy-Westropp, R.A. (1982). Studies on the hydrolysis of Vitis vinifera monoterpene precursor compounds and model monoterpene ß-D-glucosides rationalizing the monoterpene composition of grapes. J. Agric. Food Chem. 30: 1219-1223.
Wilson, B, Strauss, C.R. and Williams, P.J. (1986). The distribution of free and glycosidically bound monoterpenes among skin, juice, and pulp fractions of some white grape varieties. Am. J. Enol. Vitic. 37: 107-111.
Zeng, Y., and Yang, T. (2002). RNA isolation from highly viscous samples rich in polyphenols and polysaccharides. Plant Mol. Biol. Rep. 20: 417.

## Claims

1. Method for identifying a substrate of a glycosyl transferase of a plant, said method comprising the following steps:
a) obtaining a tissue or mixture of tissues from said plant or from a different plant that is phylogenetically closely related to said plant such that said plant and the different plant that is phylogenetically closely related to said plant have overlapping spectra of glycosyl transferase genes and glycosides;
b) obtaining from said tissue or mixture of tissues an extract comprising glycosides, said glycosides consisting of an aglycone component linked to a glycone component;
c) cleaving the glycosides comprised in the extract obtained in step b) to yield free aglycone components not linked to a glycone component, thereby forming an aglycone library;
d) carrying out a reaction of said aglycone components obtained in step c) with one or more activated sugar precursors in the presence of said glycosyl transferase to yield glycosides composed of an aglycone component linked to a glycone component;
e) identifying the glycosides obtained in step d);
wherein the aglycone components of the glycosides identified in step e) are thereby identified as substrates of said glycosyl transferase.

2. Method according to claim 1, wherein the plant tissue or mixture of plant tissues obtained in step a) shows a higher expression of the mRNA coding for said glycosyl transferase than other tissues of the same plant, preferably as detected by real-time PCR analysis, and/or a higher protein level of said glycosyl transferase than other tissues of the same plant, preferably as detected by comparative protein analysis by MALDI-TOF mass spectrometry.

3. Method according to claim 1 or 2, wherein the plant tissue or mixture of plant tissues obtained in step a) is from a developmental stage of the plant from which the plant tissue or mixture of plant tissues is obtained in which said plant tissue shows in real-time PCR analysis a higher expression of the mRNA coding for said glycosyl transferase than in other developmental stages of the same plant, and/or shows in comparative protein analysis by MALDI-TOF mass spectrometry a higher protein level of said glycosyl transferase than in other developmental stages of the same plant.

4. Method according to any of the preceding claims, wherein, prior to step a), the plant from which a tissue or mixture of tissues is obtained in step a) is exposed to an environmental stimulus, preferably an environmental stimulus selected from the group consisting of exposure to visible and/or ultra-violet light, exposure to a chemical, a less than ideal supply of water or nutrients, exposure to a pathogen or symbiont, higher or lower than standard atmospheric pressure (101325 Pa), exposure to an altered atmospheric composition compared to standard atmospheric composition, exposure to a putative glycosyl transferase substrate or one of its precursors, exposure to an enzyme inhibitor which changes substrate flow, a temperature that is higher or lower than the temperature at which the plant is commonly grown, mechanical stress, exposure to a phytohormone, preferably a phytohormone involved in pathogen or stress signaling, more preferably a phytohormone selected from the group consisting of jasmonic acid, salicylic acid, nitrogen monoxide, ethylene and a systemic peptide, exposure to a transgenic or transiently expressed regulatory protein, and exposure to a silencing RNA, wherein, preferably, said environmental stimulus results in an increase in the expression of the mRNA coding for said glycosyl transferase in said plant tissue, preferably as detected by real-time PCR analysis, and/or in an increase in the protein level of said glycosyl transferase, preferably as detected by comparative protein analysis by MALDI-TOF mass spectrometry.

5. Method according to any of the preceding claims, wherein, in step c), said cleaving the glycosides comprised in the extract is carried out by solvolysis, more preferably by thiolysis or hydrolysis, more preferably by hydrolysis.

6. Method according to any of the preceding claims, wherein, in step b), said aglycone component of said glycosides is an aliphatic or aromatic alcohol, thiol, amine or acid, preferably an aliphatic or aromatic alcohol.

7. Method according to any of the preceding claims, wherein, in step b), said glycosides have a molecular weight of below 3 000 Dalton.

8. Method according to any of the preceding claims, wherein, in step b), said glycosides are terpene glycosides, preferably mono-, sesqui- and diterpene glycosides, more preferably monoterpene glycosides.

9. Method according to any of the preceding claims, wherein in step d) a glycosyl transferase is used which is
- a recombinantly expressed glycosyl transferase or
- a preparation of said glycosyl transferase obtained by purification or partial purification from said plant, preferably from said tissue or mixture of tissues of said plant.

10. Method according to any of the preceding claims, wherein said activated sugar precursors used in step d) are selected from the group consisting of UDP-glucose, UDP-xylose, UDP-galactose, UDP-glucuronic acid, UDP-arabinose and UDP-rhamnose, wherein, preferably, said activated sugar precursor is UDP-glucose.

11. Method according to any of the preceding claims, wherein said identification in step e) is carried out by LC-MS/MS (liquid chromatography-tandem mass spectrometry), GC-MS (gas chromatography-mass spectrometry), FTIR (Fourier Transformation Infra-red Resonance) spectrometry, NMR (Nuclear Magnetic Resonance) spectroscopy, HPLC (High Performance Liquid Chromatography) or TLC (Thin Layer Chromatography).

12. Method according to any of the preceding claims, wherein said cleaving of step c) is carried out by enzymatic hydrolysis with a hydrolase or mixture of hydrolases, wherein, preferably, said hydrolase or mixture of hydrolases is selected from the group consisting of a glycosidase, a mixture of glycosidases, an esterase, a mixture of esterases, a mixture of one or more glycosidases with one or more esterases, a glucosidase and a mixture of glucosidases, wherein, more preferably said hydrolase or mixture of hydrolases is a glucosidase or a mixture of glucosidases, to yield an aglycone library prepared by enzymatic hydrolysis,
or wherein said cleaving of step c) is carried out by hydrolysis with an acid, preferably with phosphoric acid, hydrochloric acid, trichloracetic or sulphuric acid, to yield an aglycone library prepared by hydrolysis with an acid.

13. Method according to claim 12, wherein, after step c) and prior to step d), one or more of the following is done:
- said hydrolase or mixture of hydrolases is inactivated, preferably by thermic inactivation, to yield an aglycone library prepared by enzymatic hydrolysis with inactivated hydrolase(s);
- one or more inhibitors of said hydrolase(s) are added to the aglycone library prepared by enzymatic hydrolysis, to yield an aglycone library prepared by enzymatic hydrolysis including one or more inhibitors of said hydrolase(s);
- the free aglycone components obtained in step c) are separated from said hydrolase(s) to yield a purified aglycone library prepared by enzymatic hydrolysis.

14. Method according to any of the preceding claims, wherein, after step c) and prior to step d), the free aglycone components obtained in step c) are isolated from the other reaction components present during the cleaving of step c), to yield a purified aglycone library.

15. Kit comprising separately
A) a glycosyl transferase of a plant, wherein, preferably, said glycosyl transferase is a recombinantly expressed glycosyl transferase; and
B) an aglycone library as defined in any of claims 1, 12, 13 or 14; and
C) optionally, instructions for use of said aglycone library.
